# EUROPEAN PATENT APPLICATION

(11) **EP 4 231 017 A1**
(43) Date of publication of application: **23.08.2023**
(21) Application number: 22305179.8
(22) Date of filing: 17.02.2022
(51) Int. Cl.: G01N 33/68

(54) **DETECTION AND QUANTIFICATION OF ANTI-DRUG ANTIBODIES AND ANTI-SELF ANTIBODIES**

(71) Applicant: Promise Proteomics, 38000 Grenoble (FR)
(72) Inventor: CHENAU, Jérôme, 38000 GRENOBLE (FR); LEBERT, Dorothée, 38640 CLAIX (FR)
(74) Representative: Cabinet Nony

(57) **Abstract**

This invention relates to the field of antibody quantification. In particular it relates to the quantification of antibodies in a biological sample; including anti-drug antibodies (ADA), anti-self antibodies (ASA), and mixtures thereof.

## Description

### FIELD OF THE INVENTION

This invention relates to the field of antibody detection and quantification. In particular it relates to the detection and quantification of antibodies in a biological sample; including anti-drug antibodies (ADA), anti-self antibodies (ASA), and mixtures thereof.

### BACKGROUND OF THE INVENTION

The analysis of biological samples, such as plasma and serum samples, and more particularly of biological samples from humans, is of interest in the biopharmaceutical industry and for diagnosis purposes.

An increasing number of active ingredients in the pharmaceutical industry and immunotherapies is of biological origin. Among therapeutic polypeptides, peptides, proteins, antibodies and polypeptide-based drugs are particularly considered, and increasingly used as a treatment of human disease with over 3500 clinical trials ongoing worldwide. More specifically, antibody-based therapy in cancer and inflammation has become established over the past fifteen years and is now one of the most successful and important strategies for treating patients with hematological malignancies and solid tumors.

Compared to small molecules, therapeutic polypeptides show a high risk of an autoimmune response, resulting in the formation of anti-drug antibodies (ADAs), which are associated with treatment failure and can pose problem for patient safety. Currently, drug developments take into consideration this risk and improvements have been done to reduce it such as the development of fully humanized forms of therapeutic monoclonal antibodies.

Unfortunately, immunogenicity remains currently a challenge in clinical practice. The clinical effects of immune responses for patients are highly variable, ranging from no measurable effect to extremely harmful including cytokine release syndrome, cross-reactive neutralization of endogenous proteins mediating critical functions and anaphylaxis; as reported by Krishna & Nadler ("Immunogenicity to Biotherapeutics - The Role of Anti-drug Immune Complexes" (2016); Frontiers in Immunology, 7:21).

The loss of efficacy due to the presence of such ADAs includes the inactivation of the drug itself, impairment of the pharmacokinetic (PK) properties of the drug as well as its distribution, and increased clearance of ADA-drug complexes.

For example, van Brummelen et al. ("Antidrug Antibody Formation in Oncology: Clinical Relevance and Challenges"; Oncologist. 2016 (10): 1260-1268) finds that most biological anticancer drugs in a selection of clinical trials are immunogenic and induce ADAs. Such ADAs have since been detected in a wide range of drugs, including in a non-exhaustive manner those selected from murine, chimeric, humanized and human monoclonal antibodies, and protein drugs.

Apart from anti-drug antibodies, there is also a need for the detection and quantification of autoantibodies, or anti-self antibodies (ASA), which are directed against endogenous polypeptides with immunogenic properties.

Methods for detecting and quantifying antibodies such as ADAs and ASAs in biological samples, which may include serum/plasma concentration of other endogenous antibodies, shall thus be highly specific, sensitive, accurate and reproducible, in order to be applicable for clinical use.

The conventional immuno-assay based approach has been used for over 25 years and has several limitations. The immuno-assay based methods require high quality custom reagents that can take several months to generate and the assay optimization can take an additional number of months. Thus, such methods have a long assay development time which is a limitation in both the early discovery stage and the development stage of protein-based drugs. Also, suitable reagents and assay conditions may not be possible in some cases due to the highly custom binding requirements for each protein therapeutic.

Another limitation of immuno-assay based assays is that reagents may bind non-specifically to plasma/serum proteins and matrix interference is a common phenomenon. Other methods including surface plasmon resonance are also reported.

Liquid chromatography-tandem mass spectrometry (LC-MS/MS) has proven to be a reliable bioanalytical approach for the detection and quantification of both small and large molecule (such as antibodies). It offers various advantages over other techniques in terms of selectivity, linear dynamic range, method development time, enhanced precision due to the use of internal standards and multiplexing capabilities.

Methods for detecting and quantifying antibodies in a sample of an individual based on the signal emanating from labeled proteolysis peptides are, for instance, described in EP3371602B1 or EP3165922B1**.**

The direct LC-MS detection of ADAs and ASAs remains challenging because they are patient-dependent polyclonal antibodies with unknown specific sequence.

So, it is not possible to target proteotypic peptide specifics of the ADAs and ASAs with a direct strategy.

Consequently, very few methods to detect and quantify ADAs or ASAs have been reported in the Art. The two following published works have been described.

Chen et al. ("Development of Immunocapture-LC/MS Assay for Simultaneous ADA Isotyping and Semiquantitation"; Journal of Immunology Research; 2016) reports a method including a step of capturing ADAs and/or drug-ADA complexes by biotinylated drugs or anti-drug antibodies immobilized on streptavidin magnetic beads, which are then released and subjected to trypsin digestion followed by LC/MS detection of specific peptides. This method is based on the monitoring of unlabeled universal peptides. However, the proof of concept is performed for the anti-Protein Z ADAs (containing no human Fc) and, according to the authors, this approach is not applicable to molecules which contain constant human Fc regions, which is the case for therapeutic antibodies. Moreover, the authors report interferences with other endogenous immunoglobulins, thus leading to an insufficient sensitivity for more accurate ADA quantitation. The approach described in this document does not use any internal standard, and is not compatible with multiplex detection or quantification.

El Amrani et al. ("Quantification of neutralizing anti-drug antibodies and their neutralizing capacity using competitive displacement and tandem mass spectrometry: Infliximab as proof of principle"; Journal of Translational Autoimmunity; 2019..100004) reports a competitive screening assay to quantify a specific type of ADAs (neutralizing ADAs). The presence of neutralizing ADAs in patient plasma prevents the binding of labeled therapeutic antibodies to its target fixed on a solid support. However, this approach is limited to a specific type of ADAs (i.e. anti-Infliximab neutralizing ADAs). The method uses SIL-Adalimumab as an internal standard (compatible for this model as Infliximab and Adalimumab have a different recognizing site on TNFα without steric clash), but it is not compatible for multiplexed analysis.

There is thus a need for methods for the detection and quantification of ADAs and ASAs by mass spectrometry (MS), which are both suitable for multiplex quantification, and applicable to human samples in an accurate, sensitive and reproducible manner.

There is also a need for methods for the detection and quantification of ADAs and ASAs which are compatible with high-throughput studies.

In particular, there is a need for methods which are compatible with sample prefractionation steps and/or the proteolysis step that may be required before MS analysis.

The invention has for purpose to meet those needs.

### SUMMARY OF THE INVENTION

**According to a first main embodiment,** the invention relates to a method for detecting at least one anti-drug antibody (ADA) or anti-self antibody (ASA), in a sample, comprising the steps of:
a) bringing into contact a solid support conjugated to a polypeptide capable of binding to an antigen-binding site of an ADA or ASA with a test sample susceptible to contain the said ADA or ASA, in conditions suitable for the ADA or ASA to bind to the polypeptide of the solid support, whereby a solid phase is provided;
b) bringing into contact the solid support with a labeled form of the polypeptide;
c) performing enzyme proteolysis on the solid phase, or a fraction thereof, whereby a proteolysis sample susceptible to contain one or more proteotypic labeled peptide(s) from the labeled form of the polypeptide is provided;
d) detecting, by mass spectrometry analysis, the proteotypic labeled peptide(s) in the proteolysis sample, thereby detecting the ADA or ASA in the test sample.

**According to a second main embodiment,** the invention relates to a method for quantifying at least one anti-drug antibody (ADA) or anti-self antibody (ASA), in a sample, comprising the steps of:
a) bringing into contact a solid support conjugated to a polypeptide capable of binding to an antigen-binding site of an ADA or ASA with a test sample susceptible to contain the said ADA or ASA, in conditions suitable for the ADA or ASA to bind to the polypeptide of the solid support, whereby a solid phase is provided;
b) bringing into contact the solid support with a labeled form of the polypeptide;
c) performing enzyme proteolysis on the solid phase, or a fraction thereof, whereby a proteolysis sample susceptible to contain one or more proteotypic labeled peptide(s) from the labeled form of the polypeptide and one or more proteotypic unlabeled peptide(s) from the conjugated polypeptide is provided;
d) detecting, by mass spectrometry analysis, the proteotypic labeled peptide(s) and proteotypic unlabeled peptide(s) in the proteolysis sample, thereby quantifying the ADA or ASA in the test sample.

According to a **third main embodiment,** the invention relates to a kit comprising:
- a solid support conjugated to one or more drug polypeptide(s) capable of binding to an anti-drug antibody (ADA), wherein the one or more drug polypeptide(s) is selected from: a mammal antibody, a human antibody, a humanized antibody, a chimeric antibody, a synthetic antibody, a therapeutic antibody, an immunogenic polypeptide;
- a stable-isotopically labeled form of the one or more drug polypeptide(s).

According to a **fourth main embodiment,** the invention relates to a kit comprising:
- a solid support conjugated to one or more immunogenic protein(s) capable of binding to an anti-self antibody (ASA);
- a stable-isotopically labeled form of the one or more immunogenic protein(s).

### DESCRIPTION OF THE FIGURES

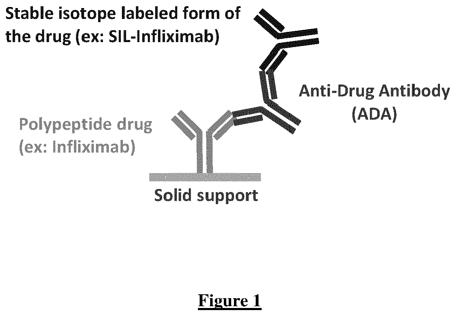

**Figure 1****: ADA Bridging LC-MS** assay. The figure depicts one intermediate complex formed during the assay, when applied to the detection and quantification of ADAs directed against a therapeutic antibody; in this case Infliximab. A therapeutic antibody (Infliximab) is conjugated to a solid support. A ternary complex is formed between the conjugated therapeutic antibody, the putative ADAs bound specifically to the therapeutic antibody and an isotopically labeled form of the therapeutic antibody (SIL).

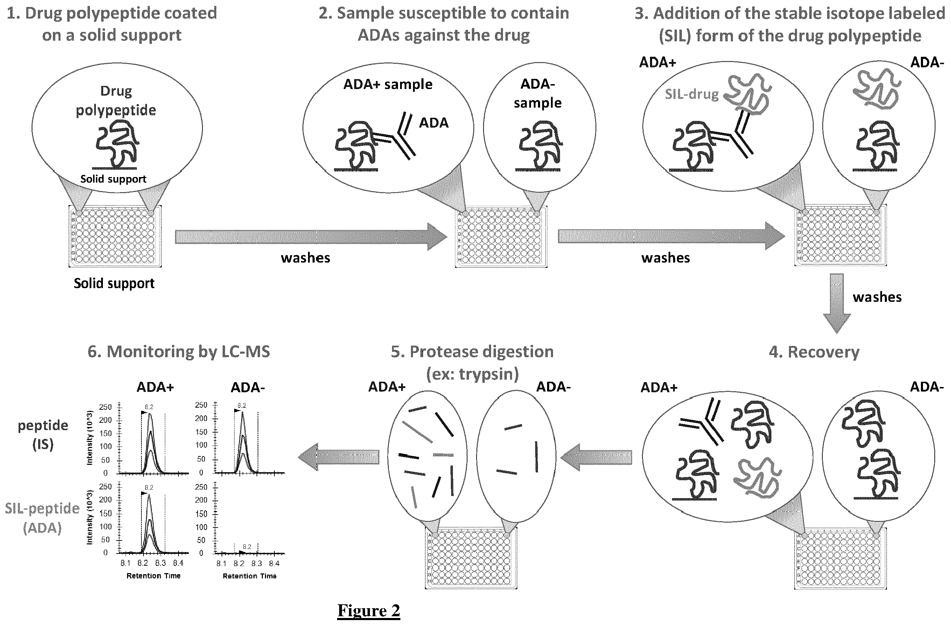

**Figure 2****: Overview of the strategy developed. Application to the quantification of anti-drug antibodies (ADAs).** The process has 6 main steps illustrated for ADAs (similar process for ASAs but directed against self polypeptide instead of drug polypeptide). 1. The drug polypeptide is immobilized on a solid support. 2. The sample susceptible to contain ADAs against the drug is added. If ADAs are present, they will recognize the drug coated on the plate. 3. The revelation is performed by the addition of the stable isotope labeled (SIL) form of the drug polypeptide. If ADAs have been bound, they will recognize the corresponding SIL-drug with their second antigen-binding site (formation of a ternary complex), otherwise they are removed during washes. 4. Recovery from the solid support of the SIL-drug, ADAs and a part of the unlabeled drug polypeptide (that can be used as an internal standard both to normalize LC-MS data and as a control for ADA negative samples). 5. Digestion with a protease resulting in a peptide mixture of the different analytes. 6. Monitoring by LC-MS of the proteotypic peptides. The SIL-peptides are only identified in samples with ADAs (formation of the ternary complex). The use of calibrators (using commercial ADAs) and the normalization with the unlabeled peptides (ratio heavy/light) allow a reliable quantification of free total ADAs (i.e. all the ADAs not bound to the putative residual drug mAb in the patient plasma). The method is applicable to all types of ADAs and ASAs, and mixtures thereof, as a uniplex or multiplex method.

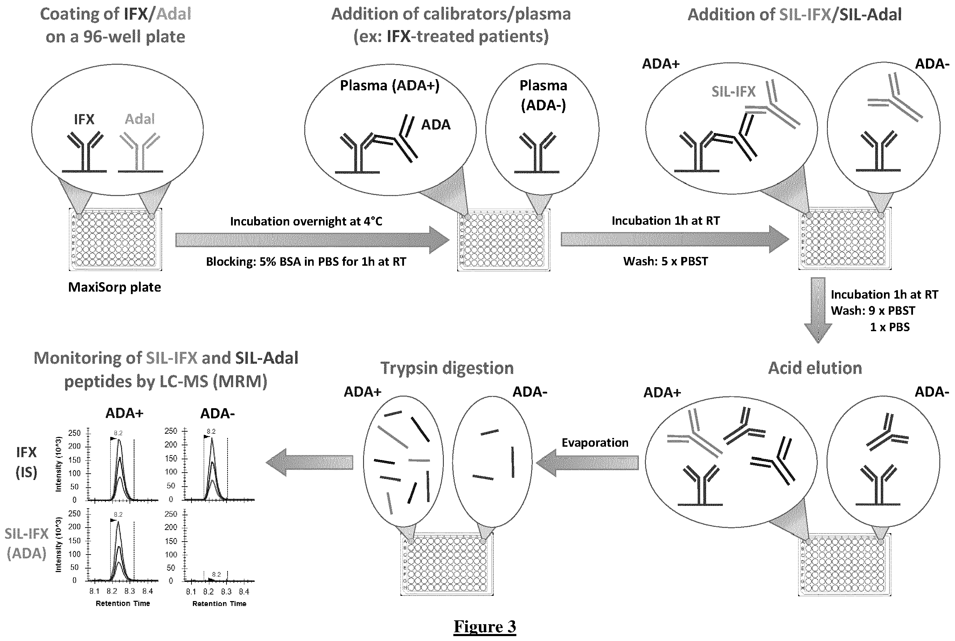

**Figure 3****: Example for the multiplex detection and quantification of anti-Infliximab and/or anti-Adalimumab anti-drug antibodies (ADAs).** Briefly, the strategy relies on the immobilization of the 2 drug mAbs (Infliximab and Adalimumab) in each well of a 96-well microplate. The sample (calibrator or patient plasma) is then added in the well. The example illustrated in this figure shows the reaction for a patient treated with Infliximab (with or without anti-Infliximab ADAs). If ADAs are present, they will recognize their antigen (i.e. the drug mAb coated on the plate). The revelation is performed by the addition of SIL-Infliximab and SIL-Adalimumab. If ADAs have been bound, they will recognize the corresponding SIL-mAb with their second antigen-binding site. An acid elution allows to release the ADAs and the SIL-mAb (only if ADAs are present). A weak release of the coated drug mAb also occurs and is used as an internal standard both to normalize LC-MS data and as a control for ADA negative samples. After trypsin digestion, proteotypic peptides are monitored by LC-MS used in MRM mode. The SIL-peptides are only identified in samples with ADAs. The use of calibrators (using commercial ADAs) and the normalization with the unlabeled peptides (ratio heavy/light) allow a reliable quantification of free total ADAs (e.g.. all the ADAs not bound to the drug mAb in the patient plasma). The method is applicable to all types of ADAs and ASAs, and mixtures thereof, as a uniplex or multiplex method.

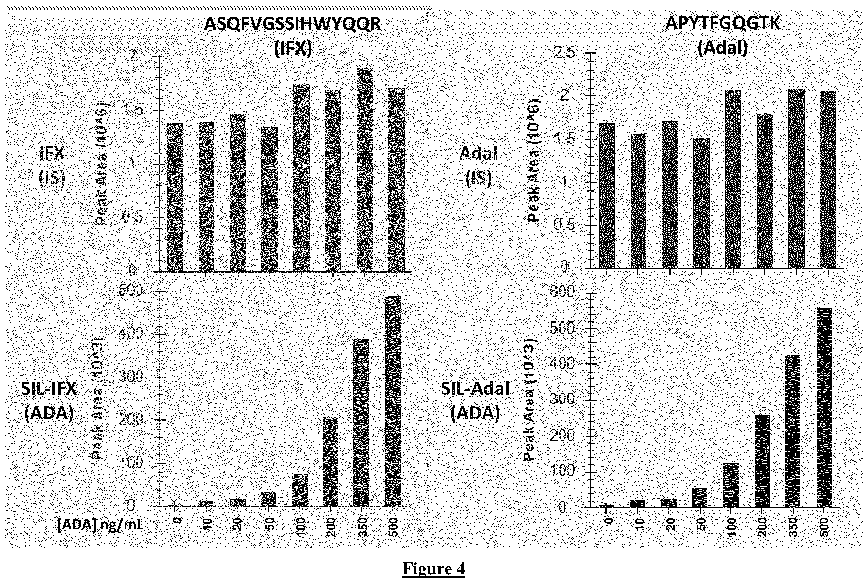

**Figure 4****: Detection & quantification of anti-Infliximab and anti-Adalimumab ADAs in human plasma.** Example of typical profiles obtained for calibrators after LC-MS analysis. Calibrators correspond to a mixture of an anti-Infliximab and anti-Adalimumab ADAs spiked at different concentrations (0, 10, 20, 50, 100, 200, 350 and 500 ng/mL) in human plasma and processed using the analytical method developed. The peak area observed for the light and the heavy (SIL: stable isotope labeled) form of 1 proteotypic peptide of Infliximab (ASQFVGSSIHWYQQR or **SEQ ID N°3)** and Adalimumab (APYTFGQGTK or **SEQ ID N°2)** is shown, respectively in the left and right panels. The light forms correspond to the weak release of the coated drug mAbs during elution and are used as an internal standard both to normalize LC-MS data (H/L ratio or Heavy/Light ratio) and as a control (digestion, LC-MS) for ADA negative samples. This release is overall stable for each well and so is a robust internal standard. The SIL-peptides are only identified in samples with ADAs with a signal directly related to the quantity of ADAs.

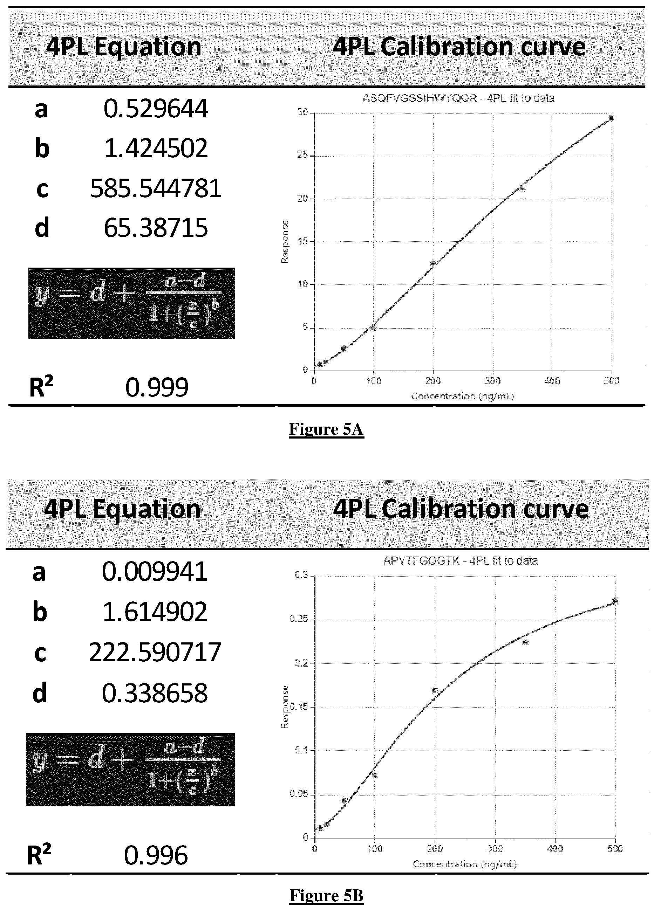

**Figure 5****: Example of calibration curves used for the quantification of anti-Infliximab and anti-Adalimumab ADAs in human plasma.** For each proteotypic peptide monitored, the H/L ratio calculated for each calibrator is used to perform the calibration curve. The calibration curve corresponding to anti-Infliximab ADAs is shown in **the A panel** and to anti-Adalimumab ADAs in the **B panel.**

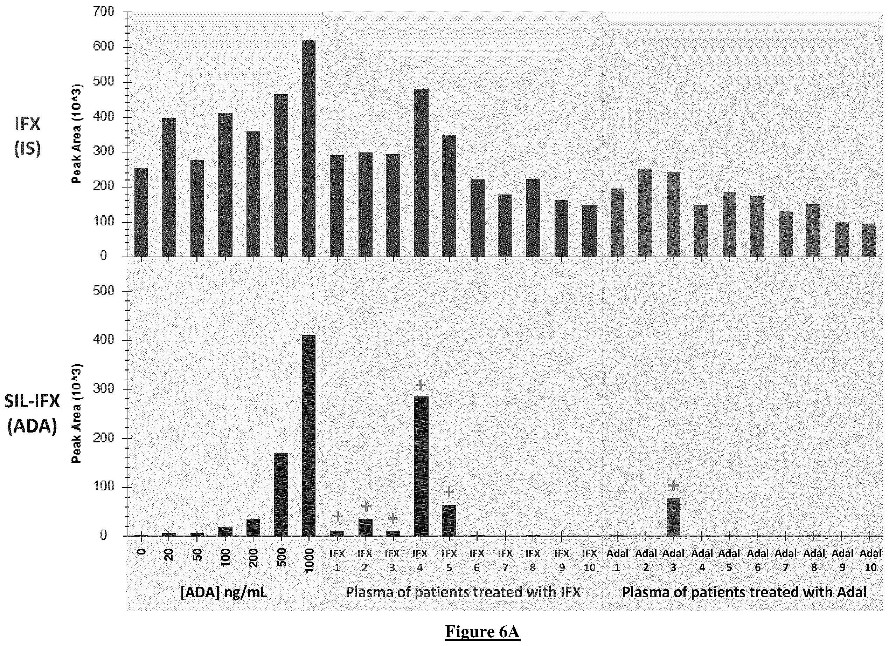

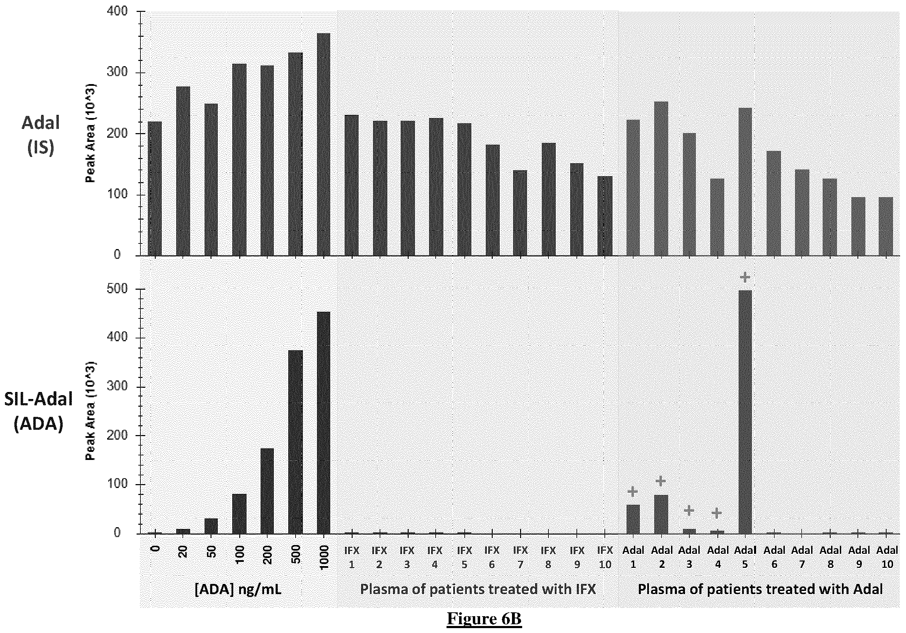

**Figure 6****: Detection & quantification of anti-Infliximab (A) or anti-Adalimumab (B) ADAs in human plasma of patient treated with Infliximab or Adalimumab using a multiplex ADA-bridging LC-MS assay.**

Data are shown here for 2 proteotypic peptides: SINSATHYAESVK (or **SEQ ID N°1)** for anti-Infliximab ADAs **(A)** and APYTFGQGTK (or **SEQ ID N°2)** for anti-Adalimumab ADAs **(B).** Each sample has been previously tested in hospital for ADAs using ELISA bridging assay and are analyzed in a blind test using the ADA-bridging LC-MS assay. **Left pane**l: calibrators from 20 to 1000 ng/mL of ADAs. **Medium panel:** patients treated with Infliximab. **Right pane**l: patients treated with Adalimumab. +: ADA-positive patient. The patient Adal 3, treated with Adalimumab, has been previously treated with Infliximab and is ADA-positive for the 2 drug mAbs.

### DETAILED DESCRIPTION OF THE INVENTION

The inventors were able to take advantage of the fact that natural - endogenous - antibodies such as ADAs and ASAs, in particular those found in mammals, possess at least two antigen binding sites that allow the formation of a ternary complex between one or more polypeptide(s), in particular drug polypeptide(s), conjugated to a solid support and a corresponding labeled form of the drug polypeptide. One example of such ternary complexes, in the context of anti-Infliximab ADAs detection, is provided in **figure 1****.**

Moreover, the inventors were then able to demonstrate, using commercial ADAs spiked at different concentrations in human plasma, that quantitative results could be obtained after binding of said ADAs to the drug polypeptide coated on a solid support, thereby forming a solid phase, and the subsequent recognition of the labeled form of the drug. Accordingly, those results were reproduced with ADAs directed against two different drugs, with a sensitivity which is similar or better to the current reference approach (ELISA assays), but with multiplex abilities. This approach has been validated on plasmas from patients previously treated with two therapeutic antibodies.

Accordingly, the inventors demonstrate that the proposed detection and quantification methods are performed by mass spectrometry analysis, and is compatible with clinical practice, as a uniplex or multiplex method.

The inventors further propose that the strategy can be applied to the detection of all protein-based drugs for which labeled form, such as a Stable-Isotopically Labeled (SIL) form, can be produced.

The methods of detection and quantification provided herein may be applied both to anti-drug antibodies (ADAs), anti-self antibodies (ASAs), and mixtures thereof. Hence, the inventors propose to adapt the methods described in **figure 2****,** which refer to methods for detecting and quantifying ADAs, ASAs, and mixtures thereof, and which may include the following steps:
(i) one or more drug polypeptide(s) (e.g. a therapeutic antibody) or antigen(s) susceptible to induce an immunogenic response is/are conjugated to a solid support;
(ii) a sample (e.g. a biological sample) susceptible to contain ADAs and/or ASAs against, respectively, the drug or the antigen is added. If ADAs or ASAs are present, they will recognize the drug or antigen conjugated to the solid support with their **first antigen-binding site** (in those examples coated on the plate);
(iii) the revelation is performed by the addition of a labeled form (e.g. a stable isotope-labeled (SIL) form) of the drug polypeptide or antigen. If ADAs or ASAs are present, they will recognize the labeled drug or labeled antigen with their **second antigen-binding site,** thereby forming a ternary complex. If ADAs or ASAs are not present the ternary complex is not formed, and the labeled drug or labeled antigen which is not part of the solid phase is removed during subsequent washes;
(iv) the labeled drug or antigen, the ADAs or ASAs susceptible to be present, and optionally all or part of the conjugated (unlabeled) drug or antigen(s) is/are recovered from the solid support, as part of the solid phase;
(v) enzyme proteolysis (e.g. trypsin proteolysis) is performed, resulting in a peptide mixture of different analytes, including proteotypic peptides.
(vi) mass spectrometry analysis (e.g. LC-MS) is performed for the monitoring of proteotypic peptides. Labeled peptides (e.g. SIL peptides) are only identified in samples with ADAs or ASAs (formation of the ternary complex)

Advantageously, the unlabeled drug or antigen which is/are optionally recovered from the solid support may further serve as internal standards, both to normalize the mass spectrometry data via a heavy/light ratio and as a control for ADA-negative or ASA-negative samples.

Advantageously, additional calibrators (e.g. commercial ADAs and ASAs) may further improve the reliability of the quantification of free total ADAs or ASAs in the sample (e.g. all the ADAs and ASAs not bound to their respective drug and antigen in the patient sample).

The method, and corresponding kits adapted to the methods, will be further developed hereafter.

**According to a first main embodiment,** the invention relates to a method for detecting at least one anti-drug antibody (ADA) or anti-self antibody (ASA), in a sample, comprising the steps of:
a) bringing into contact a solid support conjugated to a polypeptide capable of binding to an antigen-binding site of an ADA or ASA with a test sample susceptible to contain the said ADA or ASA, in conditions suitable for the ADA or ASA to bind to the polypeptide of the solid support, whereby a solid phase is provided;
b) bringing into contact the solid support with a labeled form of the polypeptide;
c) performing enzyme proteolysis on the solid phase, or a fraction thereof, whereby a proteolysis sample susceptible to contain one or more proteotypic labeled peptide(s) from the labeled form of the polypeptide is provided;
d) detecting, by mass spectrometry analysis, the proteotypic labeled peptide(s) in the proteolysis sample, thereby detecting the ADA or ASA in the test sample.

**According to a second main embodiment,** the invention relates to a method for quantifying at least one anti-drug antibody (ADA) or anti-self antibody (ASA), in a sample, comprising the steps of:
a) bringing into contact a solid support conjugated to a polypeptide capable of binding to an antigen-binding site of an ADA or ASA with a test sample susceptible to contain the said ADA or ASA, in conditions suitable for the ADA or ASA to bind to the polypeptide of the solid support, whereby a solid phase is provided;
b) bringing into contact the solid support with a labeled form of the polypeptide;
c) performing enzyme proteolysis on the solid phase, or a fraction thereof, whereby a proteolysis sample susceptible to contain one or more proteotypic labeled peptide(s) from the labeled form of the polypeptide and one or more proteotypic unlabeled peptide(s) from the conjugated polypeptide is provided;
d) detecting, by mass spectrometry analysis, the proteotypic labeled peptide(s) and proteotypic unlabeled peptides in the proteolysis sample, thereby quantifying the ADA or ASA in the test sample.

**According to said first** and **second main embodiments,** the methods according to the present disclosure may advantageously comprise a further step b2) of recovering a solid phase, or fraction thereof, containing proteotypic labeled peptides from the labeled form of the polypeptide and proteotypic unlabeled peptides from the conjugated polypeptide.

**According to said first** and **second main embodiments,** the methods according to the present disclosure may advantageously comprise a further step d2) of comparing the detected amount of proteotypic labeled peptides and proteotypic unlabeled peptides in the proteolysis sample to reference values; in particular reference values obtained from reference samples with a known amounts of ADAs or ASAs, and/or a reference sample devoid of ADAs and ASAs.

According to some preferred and examplified embodiments, the labeled form of the polypeptide is a stable-isotopically labeled form of the polypeptide.

It will be readily understood herein that the method relies on a polypeptide conjugated to a solid support, said polypeptide being capable to be recognized by the antibody (ADA, ASA, or mixture thereof) for which detection or quantification in the sample is expected. Accordingly, the method relies on the interaction between the conjugated polypeptide and the putative anti-polypeptide antibody, on one hand, and the formation of a second interface between the anti-polypeptide antibodies and the labeled form of the polypeptide.

Accordingly, the ADA, ASA or mixture thereof to be detected or quantified shall be characterized in that: the antibody comprises a plurality of (i.e. two or more) antigen binding sites for the said polypeptide, and those antigen binding sites and corresponding epitopes remain (at least in part) accessible during the formation of the solid phase.

In view of the above, it will be understood that the antibody to be detected or quantified must be suitable for the formation of the ternary complex.

It will also be understood that, in order to minimize any bias in detection and/or quantification, the conjugated polypeptide and the labeled form of the polypeptide share the same or substantially the same properties with respect to binding with the ADAs or ASAs antigen binding sites susceptible to be present in the sample; and preferably are identical, substantially identical, or share a region with polypeptide sequence identity.

According to some embodiments, the conjugated polypeptide on the solid support and the labeled form of the polypeptide share together at least 80% of polypeptide sequence identity; in particular at least 90% of sequence identity; preferably at least 99% of sequence identity.

According to some embodiments, the conjugated polypeptide on the solid support and the labeled form of the polypeptide do not possess 100% of sequence identity (i.e. are not identical).

According to some embodiments, the conjugated polypeptide on the solid support and the labeled form of the polypeptide possess 100% of sequence identity.

According to some embodiments, the method is characterized in that the polypeptide, which is capable of binding to the ADA or ASA to be detected or quantified, is selected from: an immunogenic protein, or any other polypeptide-based biological active substance suitable to generate an immune reaction and antibodies.

According to some embodiments, the method may further comprise a step of conjugating a solid support to a polypeptide that can be recognized by an ADA or ASA with a test sample susceptible to contain the said antibody, said antibody comprising a plurality of antigen binding sites for the said polypeptide, in conditions suitable for the antibody to bind to the polypeptide of the solid support.

Conjugation may consist of covalent or non-covalent binding to the solid support. When the conjugation consists of a covalent link between the drug peptide and the solid support, the covalent link may be achieved with any means known in the Art to attach a ligand to a solid support; for example the reaction of an amino group or carboxyl group to an amino-reactive or carboxyl-reactive chemical group.

According to some embodiments of the method, the drug or polypeptide is a physiological polypeptide, naturally produced by an individual.

According to some embodiments of the method, the drug or polypeptide is a fragment of the physiological polypeptide.

According to some embodiments, the method is for detecting an Anti-Self Antibody (ASA) in a sample.

According to some embodiments, the method is for quantifying an Anti-Self Antibody (ASA) in a sample.

According to some embodiments, the method is for detecting an Anti-Drug Antibody (ADA) in a sample.

According to some embodiments, the method is for quantifying an Anti-Drug Antibody (ADA), in a sample.

According to some embodiments, the method for detecting and/or quantifying an ADA or an ASA is suitable for detecting and/or quantifying multiple antibodies, namely a plurality of ADAs or ASAs, in a same experiment.

According to some embodiments, the method is thus for detecting and/or quantifying a plurality of antibodies; in particular for detecting and/or quantifying a plurality of ADAs, ASAs and mixtures thereof.

According to some particular embodiments, the method is for detecting or quantifying a plurality of ADAs, ASAs and mixtures thereof; said plurality of ADAs or ASAs being directed toward the same polypeptide, or the same fragment thereof (e.g. polyclonal ADAs from a sample patient sample).

According to some particular embodiments, the method is for detecting or quantifying ADA(s), ASA(s) and mixtures thereof; said ADA(s)s or ASA(s)s being directed toward distinct drug(s), polypeptide(s), or fragment(s) thereof.

When the method is for detecting or quantifying, in a sample ADAs or ASAs directed toward distinct drug(s), polypeptide(s), or fragment(s) thereof, the method can also be referred herein as a *"multiplex"* method.

Advantageously, the corresponding solid support may be conjugated to a plurality of distinct drug(s), polypeptides or fragments thereof; for example, the corresponding solid support may be conjugated to two or more distinct drug(s), polypeptides or fragments thereof.

Hence, according to some particular embodiments, the corresponding solid support may be conjugated to two or more distinct drug(s), polypeptides or fragments thereof; which may thus include in a non-exhaustive manner 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 60, 70, 80, 90, 100 or more distinct drug(s), polypeptides or fragments thereof.

According to some embodiments, the conjugated drug or polypeptide is a fusion protein (e.g. a fusion protein comprising a polypeptide that can induce the generation of and be recognized by one or more ADAs to be detected or quantified in the sample).

According to some embodiments, the conjugated drug or polypeptide has therapeutic activity.

According to some more particular embodiments, the conjugated drug or polypeptide is a recombinant protein suitable for use as a therapeutic agent.

According to some embodiments, the conjugated drug or polypeptide(s) is/are selected from: a mammal antibody, a human antibody, a humanized antibody, a synthetic antibody, a chimeric antibody, a therapeutic antibody; or a fragment thereof, in particular an antigen-binding fragment thereof.

According to some embodiments, the conjugated drug or polypeptide comprises at least one variable region or hypervariable region of an antibody or antigen-binding fragment; in particular a plurality of complementarity-determining regions (CDR) from an antibody or antigen-binding fragment.

According to some embodiments, the conjugated drug or polypeptide is an antibody of the immunoglobulin type selected from the IgM, IgD, IgG, IgA and IgE type; or a fragment thereof

According to some more particular embodiments, the conjugated drug or polypeptide is an antibody of the IgG type, or a fragment thereof.

According to some embodiments, the method may in particular be for detecting and/or quantifying at least one Anti-Self Antibody (ASA) in the sample, the ASA being directed against the immunogenic protein/polypeptide or other polypeptide-based biological active substance suitable to generate an immune reaction.

According to some of those embodiments, the conjugated polypeptide is an immunogenic protein/polypeptide, or any other polypeptide-based biological active substance suitable to generate an immune reaction; in particular selected from the group consisting of:
- ATG16L1, ABCF2, ABL2, TNK2, EIF2C4, APOL2, APRT, AQP2, ARL6IP4, ARHGEF1, ARHGEF16, ARIH2, ARMC1, ARMC6, ASCC2, ASF1B, ASPH, ASPHD1, ASS1, ATF1, ATG4A, IGK, AZ12,BCL2L12, BCL2L14, HSPC142, C17orf49, BCL11A, BCCIP, BCKDK, BID, BNIP3L, DC42EP4, C7orf27, BYSL, BZW2, C1orf105, C1orf131, CHAF1B, CARD9, CBS, CCDC97, CDC123, CDCA3, CDK6, CENPB, CENPQ, CEP57, VPS24, CHRAC1, C1orf77, C9orf142, CINP, CKAP2, COASY, COG3, COPZ1, CIAPIN1, RCSD1, C18orf25, CRK, CSF1R, CSNK2A1, GPS1, CTDSPL, CTNNAL1, PSCD4, DYNC1LI2, AMD1, GAD2, DCLK1, DDX19B, DDX21, DDX54, DEF6, DGCR8, DHX8, DMD, DNAJC8, LIG3, C19orf26, DPM1, POLE, DPYSL3, DPYSL4, DPYSL5, DTNBP1, DTL, DDX39A, DYNC1H1, ECE1, EIF3S4, ELOVL5, EME1, C11orf30, EPM2AIP1, ETS1, ETS2, FAM125A, MGC40405, C14orf24, C9orf25, FAM63B, FKBP1A, WTAP, FMNL2, FMR1, FKSG44, FYN, GPI, TUBGCP3, SIP1, GGA1,GSK3B, HIST2H2BE, HIST3H2BB, UCHL5IP, HCK, HDAC9, HMGB1, HMGB2, HNRNPK, HSF1, EIF4H, IGF1R, IMPDH2, IFNGR2, ITM2C, JAK1, JAK3, C22orf9, C12orf41, PRKAR2B, CSNK1G3, KDM2A, JMJD2D, KLC2, KLC3, KLHDC4, KLF3, RPS6KA6, RPS6KB2, LARP2, LARP1, LARP4, LARP7, LUC7L2, LCK, LRBA, LRRFIP1, MAP3K2, MAP4K4, MAN2C1, MAGEB2, MAPKAPK2, MARK1, MAX, MBIP, MCF2L, MCM2, MCM6, MED1, MED4, C2orf33, FLJ20323, MAPK6, MAPK13, MKNK2, MAP2K3, CDC42BPA, MSL3L1, MST4, CBFA2T2, MTMR2, MTMR14, MYLK, NADK, NCF1, NFIC, ZCCHC17, NOL7, HSPC111, NPM1, NUP133, NUDT9, OSBPL11, OSBPL1A, PIK3C2A, PCYT1B, PDCD4, PELO, PGM1, PGM2, PLEKHA3, LCP1, PNPT1, PPP1R14A, PSMC4, PSIP1, PSMD4, PSRC1, PTEN, PTPN18, RAB3IP, RAB9A, RASSF3, hCG_20114, RBM39, RBM4B, RAB11FIP2, RING1, RIOK1, RPAIN, RPL12, RSL1D1, RPL30, RPL31, RNF138, ELAC2, ROCK1, POLR2I, POLR3C, RSRC2, MRPS18B, SNRPA1, RUSC1, RUVBL2, SLC23A2, SAE2, SAMHD1, CIP29, SCLT1, SCML1, SELPLG, SENP2, SEPP1, SF3B2, SH2B1, SIN3A, SKIL, SMARCA5, RY1, USP39, SNX1, SP100, SPAG6, SPC24, CCDC99, APEG1, SPHK2, SQSTM1, SFRS6, SSH3, STK33, STK4, STRAP, TSC22D3, GTF2F1, MAP3K7IP1, TAGLN2, TALDO1, TBC1D22A, TBCB, TCF19, SUB1, CCT8, TNS1, TGFBR2, TTN, TPD52L2, TPI1, TRDN, TRPT1, TRPV2, TSGA10, TTC1, TEX264, TXNL4A, TYK2, TYMS, UBE2O, USP15, UCK2, VDP, VAMP4, VAPB, VPS37A, WWC2, WWP2, YES1, ZAP70, LOC730411, CCDC131, ZC3H3, ZHX1, ZNF174, ZNF326, ZRANB2, ANPEP, CCDC90B, DDX43, DPP3, ERR beta-LBD, EXOC5, FTH1, GAGE1, IL10RB, KCNIP2, LSM12, MAP3K14, MAPK14, RUNX1T1, MGC42105, NRBP1, PHYHD1, PLSCR1, RNF7, SGK2, TBN, TAF9B, TPM3, TTYH2, OGN, PIR, FNTB, SERPINB3, LSM3, CSE1L, CSTB, IDH1, TRUB1, ATIC, RFX5, METTL9, S100A7A, SUMO2, PTBP2, ALDOB, GAGE1, CRKL, UBQLN1, EIF1, COQ3, PPP2R1B, GSDML, IL1F8, CTBP2, UBE2V1, GAD1, NUDCD1, UNC119, YKT6, ODF3L2, RWDD4A, GGA3, HPRT1, TCEAL5, SKP1A, ZADH1, AHSA1, PSME2, AGPAT1, C5orf15, ZBED1, C17orf62, UBE2S, RSU1, RAB3IL1, SDF4, ANXA5, PABPC5, SORD, PSMD10, C3orf26, NCF2, UCK1, PITPNB, WARS, DUSP12, COG8, LAIR1, CASP3, AKR1B 10, COQ9, HOXA1, THOP1, GSN, UBA3, GABRA6, SLC22A7, RRM1, EIF2C1, GCC1, USP4, FAM13A1, PFKL, SF3B3, GMFB, C7orf16, MOK, FGF2, aa1-155, SERPINA3, APTX, ARNTL, C20orf43, CYFIP2, CYGB, EIF2B1, FLOT2, GIMAP5, GMDS, HK1, MAGEA4, MAGEA10, MLX, MPP3, NACA2, CYB5R4, NECAP2, PANK1, PDE9A, PIGG, PITPNA, PLCD1, POU5F1, PTMS, DSCR1, RPL22, MRPS27, SAP18, DNTT, TGM4, VSNL1, VPS26A, RCHY1, ZSCAN21, and/or
- Ro-RNP complex, La antigen, Small nuclear RNP, Histones, Topoisomerase I, Centromere, Myeloperoxidase (p-ANCA), proteinase 3 (c-ANCA), Cardiolipin, Citrullinated protein, Carbamylated protein, Rheumatoid Factor (RF), Collagen, Type IV collagen, and/or

SSA, SSB, Ro52, Ro60, La, RNA-binding proteins, RNP-A, U1-70K, Sm-D3, Jo1, PL7, threonyl t-RNA synthetase, PL-12 alanyl-tRNA synthetase, Mi2 histone acetylase, tRNA synthetase proteins, TIF-γ, MDA-5, 3-hydroxy-3-methylglutaryl-coenzyme A reductase, centromere proteins, CENPA, CENPB, PM/SCL, EXOSC9, EXOS107, RNA polymerase 3 complex, POLR3A, POLR3K, insulin, IA-2, glutamate decarboxylase/GAD65, Znt8, tetraspanin-7, peptidylpropyl isomerase like 2, Mlhl, proteinase-3, myeloperoxidase, nicotinic acetylcholine receptor/nAChR, muscle-associated cell surface tyrosine kinase, low-density lipoprotein receptor-related protein, aquaporin-4, myelin oligodendrocyte glycoprotein, N-methyl-D-asparate receptor/NMDAR, alpha-amino-3-hydroxy-5methyl-4-isoxazolepropionic receptor/AMPAR, leucine rich glioma inactivated 1 protein, neurexin family protein contactin associated protein 2, phospholipase A2 receptor, thrombospondin type-1 domain-containing 7A, GMCSF, interferon-y, CHRNA1, LRP4, GPIHBP1, GluRl, ADAMTS13, FGF23, CSF2RA, IFNGR, PLA2R; and fragments thereof.

According to some embodiments, the method may in particular be for detecting and/or quantifying at least one Anti-Drug Antibody (ADA), wherein the drug is a therapeutic antibody and/or a fusion protein and/or a therapeutic protein.

According to some of those embodiments, the conjugated drug or polypeptide is a therapeutic antibody or fusion protein or therapeutic protein, in particular a drug monoclonal antibody (drug mAb), selected from the group consisting of: 3F8, Abagovomab, Abatacept, Abciximab, Abituzumab, Abrezekimab, Abrilumab, Actoxumab, Adalimumab, Adecatumumab, Ado-trastuzumab emtansine, Aducanumab, Aflibercept, Afutuzymab, Afasevikumab, Afelimomab, Alacizumab, Alefacept pegol, Alemtuzumab, Alirocumab, Altumomab, Amatixumab pentetate, Amatuximab, Amivantamab, Anatumomab mafenatox, Andecaliximab, Anetumab, Anifromumab ravtansine, Anifrolumab, Ansuvimab, Anrukinzumab, Apolizumab, Aprutumab ixadotin, Arcitumomab, Ascrinvacumab, Aselizumab, Atezolizumab, Atidortoxumab, Atinumab, Altizumab, Atoltivimab, Atorolimumab, Avelumab, Azintuxizumab vedotin, Bamlanivimab, Bapineuzumab, Basiliximab, Bavituximab, BCD-100, Bectumomab, Begelomab, Belatacept, Belantamab mafodotin, Belimumab, Bemarituzumab, Benralizumab, Berlimatoxumab, Bermekimab, Bersanlimab, Bertilimumab, Besilesomab, Bevacizumab, Bezlotoxumab, Biciromab, Bimagrumab, Bimekizumab, Birtamimab, Bivatuzumab, Bleselumab, Blinatumomab, Blontuvetmab, Blosozumab, Bococizumab, Brazikumab, Brentuximab vedotin, Briakinumab, Brodalumab, Brolucizumab, Brontictuzumab, Burosumab, Cabiralizumab, Camidanlumab tesirine, Camrelizumab, Canakinumab, Cantuzumab mertansine, Cantuzumab ravtansine, Caplacizumab, Casirivimab, Capromab, Carlumab, Carotuximab, Catumaxomab, cBR96-doxorubicin immunoconjugate, Cedelizumab, Cemiplimab, Cergutuzumab amunaleukin, Certolizumab pegol, Cetuximab, Cetrelimab, Cetuximab, Cibisatamab, Cirmtuzumab, Citatuzumab bogatox, Cixutumumab, Clazakizumab, Clenoliximab, Clivatuzumab tetraxetan, Codrituzumab, Cofetuzumab pelidotin, Coltuximab ravtansine, Conatumumab, Concizumab, Cosfroviximab, Crenezumab, Crizanlizumab, Crotedumab, CR6261, Cusatuzumab, Dacetuzumab, Daclizumab, Dalotuzumab, Dapirolizumab pegol, Daratumumab, Dectrekumab, Demcizumab, Denintuzumab mafodotin, Denosumab, Depatuxizumab mafodotin, Derlotuximab biotin, Detumomab, Dezamizumab, Dinutuximab, Dinutuximab beta, Diridavumab, Domagrozumab, Dorlimomab aritox, Dostarlimab, Drozitumab, DS-8201, Duligotuzumab, Dupilumab, Durvalumab, Dusigitumab, Duvortuxizumab, Ecromeximab, Eculizumab, Edobacomab, Edrecolomab, Efalizumab, Efungumab, Eldelumab, Elezanumab, Elgemtumab, Elotuzumab, Elsilimomab, Emactuzumab, Emapalumab, Emibetuzumab, Emicizumab, Enapotamab vedotin, Enavatuzumab, Enfortumab vedotin, Enlimomab pegol, Enoblituzumab, Enokizumab, Enoticumab, Ensituximab, Epcoritamab, Epitumomab cituxetan, Epratuzumab, Eptinezumab, Erenumab, Erlizumab, Ertumaxomab, Etanercept, Etaracizumab, Etesevimab, Etigilimab, Etrolizumab, Evinacumab, Evolocumab, Exbivirumab, [fam-]trastuzumab deruxtecan, Fanolesomab, Faralimomab, Faricimab, Farletuzumab, Fasinumab, FBTA05, Felvizumab, Fezakinumab, Fibatuzumab, Ficlatuzumab, Figitumumab, Firivumab, Flanvotumab, Fletikumab, Flotetuzumab, Fontolizumab, Foralumab, Foravirumab, Fremanezumab, Fresolimumab, Frovocimab, Frunevetmab, Fulranumab, Futuximab, Galcanezumab, Galiximab, Gancotamab, Ganitumab, Gantenerumab, Gatipotuzumab, Gavilimomab, Gedivumab, Gemtuzumab ozogamicin, Gevokizumab, Gilvetmab, Gimsilumab, Girentuximab, Glembatumumab vedotin, Golimumab, Gomiliximab, Gosuranemab, Guselkumab, Ianalumab, Ibalizumab, Sintilimab, Ibritumomab tiuxetan, Icrucumab, Idarucizumab, Ifabotuzumab, Igovomab, Iladatuzumab vedotin, Imalumab, Imaprelimab, Imciromab, Imdevimab, Imgatuzumab, Inclacumab, Indatuximab ravtansine, Indusatumab vedotin, Inebilizumab, Infliximab, Intetumumab, Inolimomab, Inotuzumab ozogamicin, Ipilimumab, Iomab-B, Iratumumab, Isatuximab, Iscalimab, Istiratumab, Itolizumab, Ixekizumab, Keliximab, Labetuzumab, Lacnotuzumab, Ladiratuzumab vedotin, Lampalizumab, Lanadelumab, Landogrozumab, Laprituximab emtansine, Larcaviximab, Lebrikizumab, Lecanemab, Lemalesomab, Lendalizumab, Lenvervimab, Lenzilumab, Lerdelimumab, Leronlimab, Lesofavumab, Letolizumab, Lexatumumab, Libivirumab, Lifastuzumab vedotin, Ligelizumab, Loncastuximab tesirine, Losatuxizumab vedotin, Lilotomab satetraxetan, Lintuzumab, Lirilumab, Lodelcizumab, Lokivetmab, Lorvotuzumab mertansine, Lucatumumab, Lulizumab pegol, Lumiliximab, Lumretuzumab, Lupartumab, Lupartumab amadotin, Lutikizumab, Maftivimab, Mapatumumab, Margetuximab, Marstacimab, Maslimomab, Mavrilimumab, Matuzumab, Mepolizumab, Metelimumab, Milatuzumab, Minretumomab, Mirikizumab, Mirvetuximab soravtansine, Mitumomab, Modotuximab, Mogamulizumab, Monalizumab, Morolimumab, Mosunetuzumab, Motavizumab, Moxetumomab pasudotox, Muromonab-CD3, Nacolomab tafenatox, Namilumab, Naptumomab estafenatox, Naratuximab emtansine, Narnatumab, Narsoplimab, Natalizumab, Navicixizumab, Navivumab, Naxitamab, Nebacumab, Necitumumab, Nemolizumab, NEOD001, Nerelimomab, Nesvacumab,Netakimab, Nimotuzumab, Nirsevimab, Nivolumab, Nofetumomab merpentan, Obiltoxaximab, Obinutuzumab, Ocaratuzumab, Ocrelizumab, Odesivimab, Odulimomab, Ofatumumab, Olaratumab, Oleclumab, Olendalizumab, Olokizumab, Omalizumab, Omburtamab, OMS721, Onartuzumab, Ontuxizumab, Onvatilimab, Opicinumab, Oportuzumab monatox, Oregovomab, Orticumab, Otelixizumab, Otilimab, Otlertuzumab, Oxelumab, Ozanezumab, Ozoralizumab, Pagibaximab, Palivizumab, Pamrevlumab, Panitumumab, Pankomab, Panobacumab, Parsatuzumab, Pascolizumab, Pasotuxizumab, Pateclizumab, Patritumab, PDR001, Pembrolizumab, Pemtumomab, Penpulimab, Perakizumab, Pertuzumab, Pexelizumab, Pidilizumab, Pinatuzumab vedotin, Pintumomab, Placulumab, Prezalumab, Plozalizumab, Pogalizumab, Polatuzumab vedotin, Ponezumab, Porgaviximab, Prasinezumab, Prezalizumab, Priliximab, Pritoxaximab, Pritumumab, PRO 140, Quilizumab, Racotumomab, Radretumab, Rafivirumab, Ralpancizumab, Ramucirumab, Ranevetmab, Ranibizumab, Raxibacumab, Ravagalimab, Ravulizumab, Refanezumab, Regavirumab, Regdanvimab, Relatlimab, Remtolumab, Reslizumab, Retifanlimab, Rilonacept, Rilotumumab, Rinucumab, Risankizumab, Rituximab, Rivabazumab pegol, Robatumumab, Rmab, Roledumab, Romilkimab, Romosozumab, Rontalizumab, Rosmantuzumab, Rovalpituzumab tesirine, Rovelizumab, Rozanolixizumab, Ruplizumab, SA237, Sacituzumab govitecan, Samalizumab, Samrotamab vedotin, Sarilumab, Satralizumab, Satumomab pendetide, Secukinumab, Selicrelumab, Seribantumab, Setoxaximab, Setrusumab, Sevirumab, Sibrotuzumab, SGN-CD19A, SHP647, Sifalimumab, Siltuximab, Simtuzumab, Siplizumab, Sirtratumab vedotin, Sirukumab, Sofituzumab vedotin, Solanezumab, Solitomab, Sonepcizumab, Sontuzumab, Sotrovimab, Spartalizumab, Spesolimab, Stamulumab, Sulesomab, Suptavumab, Sutimlimab, Suvizumab, Suvratoxumab, Tabalumab, Tacatuzumab tetraxetan, Tadocizumab, Tafasitamab, Talacotuzumab, Talizumab, Talquetamab, Tamtuvetmab, Tanezumab, Taplitumomab paptox, Tarextumab, Tavolimab, Tebentafusp, Teclistamab, Tefibazumab, Telimomab aritox, Telisotuzumab, Telisotuzumab vedotin, Tenatumomab, Teneliximab, Teplizumab, Tepoditamab, Teprotumumab, Tesidolumab, TGN 1412, Tetulomab, Tezepelumab, TGN1412, Tibulizumab, Tildrakizumab, Tigatuzumab, Timigutuzumab, Timolumab, tiragolumab, Tiragotumab, Tislelizumab, Tisotumab vedotin, TNX-650, Tocilizumab, Tomuzotuximab, Toralizumab, Toripalimab, Tosatoxumab, Tositumomab, Tovetumab, Tralokinumab, Trastuzumab, Trastuzumab duocarmazine, Trastuzumab emtansine, TRBS07, Tregalizumab, Tremelimumab, Trevogrumab, Tucotuzumab celmoleukin, Tuvirumab, Ublituximab, Ulocuplumab, Urelumab, Urtoxazumab, Ustekinumab, Utomilumab, Vadastuximab talirine, Vanalimab, Vandortuzumab vedotin, Vantictumab, Vanucizumab, Vapaliximab, Varisacumab, Varlilumab, Vatelizumab, Vedolizumab, Veltuzumab, Vepalimomab, Vesencumab, Visilizumab, Vobarilizumab, Volociximab, Vonlerolizumab, Vopratelimab, Vorsetuzumab mafodotin, Votumumab, Vunakizumab, Xentuzumab, XMAB-5574, Zalutumumab, Zanolimumab, Zatuximab, Zenocutuzumab, Ziralimumab, Zolbetuximab, Zolimomab aritox; and fragments thereof.

According to some embodiments, the conjugated drug or polypeptide is selected from the group consisting of: Abarelix, Abatacept, Abciximab, Adalimumab, Aflibercept, Agalsidase beta, Albiglutide, Aldesleukin, Alefacept, Alemtuzumab, Alglucerase, Alglucosidase alfa, Alirocumab, Aliskiren, Alpha-1-proteinase inhibitor, Alteplase, Anakinra, Ancestim, Anistreplase, Anthrax immune globulin human, Antihemophilic Factor, Anti-inhibitor coagulant complex, Antithrombin Alfa, Antithrombin III human, Antithymocyte globulin, Aprotinin, Arcitumomab, Asfotase Alfa, Asparaginase, Asparaginase erwinia chrysanthemi, Atezolizumab, Basiliximab, Becaplermin, Belatacept, Belimumab, Beractant, Bevacizumab, Bivalirudin, Blinatumomab, Botulinum Toxin Type A, Botulinum Toxin Type B, Brentuximab vedotin, Brodalumab, Buserelin, C1 Esterase Inhibitor, Canakinumab, Capromab, Certolizumab pegol, Cetuximab, Choriogonadotropin alfa, Chorionic Gonadotropin, Coagulation factor ix, Coagulation factor VIIa, Coagulation factor X human, Coagulation Factor XIII A-Subunit (Recombinant), Collagenase, Conestat alfa, Corticotropin, Cosyntropin, Daclizumab, Daptomycin, Daratumumab, Darbepoetin alfa, Defibrotide, Denileukin diftitox, Denosumab, Desirudin, Digoxin Immune Fab, Dinutuximab, Dornase alfa, Drotrecogin alfa, Dulaglutide, Eculizumab, Efalizumab, Efmoroctocog alfa, Elosulfase alfa, Elotuzumab, Enfuvirtide, Epoetin alfa, Epoetin zeta, Eptifibatide, Etanercept, Evolocumab, Exenatide, Factor IX Complex (Human), Fibrinogen Concentrate (Human), Fibrinolysin aka plasmin, Filgrastim, Filgrastim-sndz, Follitropin alpha, Follitropin beta, Galsulfase, Gastric intrinsic factor, Gemtuzumab ozogamicin, Glatiramer acetate, Glucagon recombinant, Glucarpidase, Golimumab, Gramicidin D, Hepatitis A Vaccine, Hepatitis B immune globulin, Human calcitonin, Human clostridium tetani toxoid immune globulin, Human rabies virus immune globulin, Human Rho(D) immune globulin, Human Serum Albumin, Human Varicella-Zoster Immune Globulin, Hyaluronidase, Ibritumomab, Ibritumomab tiuxetan, Idarucizumab, Idursulfase, Imiglucerase, Immune Globulin Human, Infliximab, Insulin aspart, Insulin Beef, Insulin Degludec, Insulin detemir, Insulin Glargine, Insulin glulisine, Insulin Lispro, Insulin Pork, Insulin Regular, Insulin porcine, Insulin isophane, Interferon Alfa-2a, Interferon alfa-2b, Interferon alfacon-1, Interferon alfa-nl, Interferon alfa-n3, Interferon beta-la, Interferon beta-lb, Interferon gamma-lb, Intravenous Immunoglobulin, Ipilimumab, Ixekizumab, Laronidase, Lenograstim, Lepirudin, Leuprolide, Liraglutide, Lucinactant, Lutropin alfa, Mecasermin, Menotropins, Mepolizumab, Methoxy polyethylene glycol-epoetin beta, Metreleptin, Muromonab, Natalizumab, Natural alpha interferon, Necitumumab, Nesiritide, Nivolumab, Obiltoxaximab, Obinutuzumab, Ocriplasmin, Ofatumumab, Omalizumab, Oprelvekin, OspA lipoprotein, Oxytocin, Palifermin, Palivizumab, Pancrelipase, Panitumumab, Pegademase bovine, Pegaptanib, Pegaspargase, Pegfilgrastim, Peginterferon alfa-2a, Peginterferon alfa-2b, Peginterferon beta-la, Pegloticase, Pegvisomant, Pembrolizumab, Pertuzumab, Poractant alfa, Pramlintide, Preotact, Protamine sulfate, Protein S human, Prothrombin complex concentrate, Ragweed Pollen Extract, Ramucirumab, Ranibizumab, Rasburicase, Raxibacumab, Reteplase, Rilonacept, Rituximab, Romiplostim, Sacrosidase, Salmon Calcitonin, Sargramostim, Satumomab Pendetide, Sebelipase alfa, Secretin, Secukinumab, Sermorelin, Serum albumin, Serum albumin iodonated, Siltuximab, Simoctocog Alfa, Sipuleucel-T, Somatotropin, Somatropin, Streptokinase, Sulodexide, Susoctocog alfa, Taliglucerase alfa, Teduglutide, Teicoplanin, Tenecteplase, Teriparatide, Tesamorelin, Thrombomodulin Alfa, Thymalfasin, Thyroglobulin, Thyrotropin Alfa, Tocilizumab, Tositumomab, Trastuzumab, Tuberculin Purified Protein Derivative, Turoctocog alfa, Urofollitropin, Urokinase, Ustekinumab, Vasopressin, Vedolizumab, Velaglucerase alfa ; and fragments thereof.

According to some embodiments, the method may in particular be for detecting and/or quantifying at least one Anti-Drug Antibody (ADA) directed toward any one of the above-mentioned drug or polypeptide.

According to some embodiments, the test sample (e.g. the biological sample) is selected from: a tissue sample, a biofluid (e.g. cerebrospinal fluid, urine, saliva..), a whole blood sample, a plasma sample and a serum sample; in particular a plasma sample.

According to some embodiments, the solid support is selected from: beads, particles, fibers, plates, gels and resins.

According to some embodiments, the polypeptide is bound covalently to the solid support.

According to some embodiments, the polypeptide is bound non-covalently to the solid support.

According to preferred embodiments of the method for quantifying ADAs and ASAs, the polypeptide is bound non-covalently to the solid support.

According to some embodiments, the method comprises a step of bringing into contact a solid support conjugated to a plurality of distinct polypeptides capable of binding to one or more ADAs or ASAs with a test sample susceptible to contain the said ADAs and ASAs.

According to some embodiments, the solid support is conjugated to a plurality of distinct polypeptides capable of binding to one or more ADAs or ASAs which are susceptible to be present in the test sample.

When the solid support is conjugated to a plurality of distinct polypeptides, the plurality of said polypeptides may, in particular, be chosen from any selection of the recited antibodies and immunogenic proteins/polypeptides, or fragments thereof, within the instant disclosure.

According to some embodiments, the enzyme proteolysis is a trypsin proteolysis.

According to some embodiments, the method comprises a step of discarding antibodies from the test sample, in particular ADAs and/or ASAs from the test sample which is/are not part of the solid phase.

According to some embodiments, the method comprises a step of discarding the fraction of the labeled form of the polypeptide which is not part of the solid phase.

According to some embodiments, the method comprises a step of recovering all or part of the solid phase by an acid treatment.

Kits suitable for the methods of detection and quantification according to the present disclosure, as defined above, are thus further considered hereafter as main embodiments.

According to a **third main embodiment,** the invention relates to a kit comprising:
- a solid support conjugated to one or more drug polypeptide(s) capable of binding to an anti-drug antibody (ADA), wherein the one or more drug polypeptide(s) is selected from: a mammal antibody, a human antibody, a humanized antibody, a chimeric antibody, a synthetic antibody, a therapeutic antibody, an immunogenic polypeptide;
- a stable-isotopically labeled form of the one or more drug polypeptide(s).

According to some particular embodiments, the invention relates to a kit comprising:
- a solid support conjugated to one or more drug polypeptides capable of binding to an anti-drug antibody (ADA), wherein the drug polypeptides are selected from: a mammal antibody, a human antibody, a humanized antibody, a chimeric antibody, a synthetic antibody, a therapeutic antibody;
- a stable-isotopically labeled form of the polypeptides.

According to some particular embodiments, the invention relates to a kit comprising:
- a solid support conjugated to a plurality of distinct drug polypeptides capable of binding to an anti-drug antibody (ADA), wherein the drug polypeptides are selected from: a mammal antibody, a human antibody, a humanized antibody, a chimeric antibody, a synthetic antibody, a therapeutic antibody, an immunogenic polypeptide;
- a stable-isotopically labeled form of the polypeptides.

According to a **fourth main embodiment,** the invention relates to a kit comprising:
- a solid support conjugated to one or more immunogenic polypeptide(s) capable of binding to an anti-self antibody (ASA);
- a stable-isotopically labeled form of the one or more immunogenic polypeptide(s).

According to some particular embodiments, the invention relates to a kit comprising:
- a solid support conjugated to a plurality of distinct immunogenic polypeptides capable of binding to an anti-self antibody (ASA);
- a stable-isotopically labeled form of the immunogenic polypeptides.

The said kits may contain the reagents and consumables required to prepare patient samples with the goal to detect and/or quantify ADAs or ASAs.

The kits which are described herein are particularly adapted to the methods of detection and quantification of ADAs, when the ADAs to be detected or quantified is directed against a therapeutic antibody.

According to some particular embodiments, the said kit may further comprise one or more calibrators, in particular suitable for setting up calibration curves.

Such calibrators may, for example, consist of one or more compositions with known amounts of ADAs or ASAs, or fragments thereof (e.g. commercial ADAs and ASAs).

According to some particular embodiments, the said kit may thus further comprise one or more antibodies, e.g. isolated antibodies, or compositions thereof, selected from: an anti-drug antibody (ADA), an anti-self antibody (ASA).

According to some particular embodiments, the said solid support from the kit may be conjugated to a plurality of distinct drug or polypeptides capable of binding to anti-drug antibodies (ADAs), wherein the drug is selected from: a mammal antibody, a human antibody, a humanized antibody, a chimeric antibody, a synthetic antibody, a therapeutic antibody, an immunogenic polypeptide, a fusion protein, a therapeutic protein.

According to some particular embodiments, the said kit may further comprise one or more enzyme proteases; for example trypsin.

### Definitions

As used herein, the expression *"detection"* or *"detecting"* refers to the identification of a presence of the ADA or ASA which is considered for detection in a given sample. Such detection may further encompass a quantification of the amount of the corresponding ADA or ASA, but not necessarily. Accordingly, the expression *"quantification"* or *"quantifying"* includes a determination of an amount of the ADA or ASA in the sample, either in relative terms (e.g. a fold change of the quantity compared to a reference), or in absolute terms (e.g. the precise quantity in a sample or a fraction thereof).

As used herein, the expression *"comprises"* or *"comprising"* encompasses also *"consists of"* or *"consisting of".*

As used herein, the expression *"a"* or *"at least one"* encompasses *"one",* or *"more than one"*; which encompasses a *"plurality",* such as two, or more than two, which may encompass, three, four, five, six or even more than six.

As used herein, and unless instructed otherwise, the term *"**at least one"*** may thus encompass *"one or more",* or even *"two or more"* (or *"a plurality"*)*.* For instance, it may encompass 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, or more than 100.

As used herein, and unless instructed otherwise, the term *"less than..."* may encompass all values from 0 to the corresponding threshold, For instance, it may encompass less than 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, or less than 100, when applicable.

As used herein, a *"labeled"* polypeptide, such as a labeled therapeutic antibody, also referred herein as the "labeled form of therapeutic antibody", consists of an antibody having the same or substantially the same amino acid sequence as a polypeptide against which the ADA or ASA is presumed to bind, which may for example be a therapeutic antibody, and which has been obtained by a method according to which one or more isotopically labeled amino acids have been incorporated in the polypeptide chain(s).

As used herein, an *"immune response"* or *"immunity"* or *"immunogenicity"* as the terms are interchangeably used herein, is meant the induction of a humoral (i.e., B cell) and/or cellular (i.e., T cell) response. Suitably, a humoral immune response may be assessed by measuring the antigen-specific antibodies present in serum of immunized animals in response to introduction of the antigen into the host. The immune response may be assessed by the enzyme linked immunosorbant assay of sera of immunized mammals, or by microneutralization assay of immunized animal sera. A CTL assay can be employed to measure the T cell response from lymphocytes isolated from the spleen or other organs of immunized animals. Accordingly, the conjugated polypeptide(s) which are considered for the methods and kits of the present disclosure may comprise or consist of an immunogenic protein, or any other polypeptide-based biological active substance suitable to generate an immune reaction.

As used herein, the term *"antibody"* or *"immunoglobulin"* have the same meaning, and will be used equally in the present invention. The term *"antibody"* as used herein refers to folded immunoglobulin molecules and immunologically active portions of immunoglobulin molecules, i.e., molecules that contain an antigen binding site that immunospecifically binds an antigen. As such, and unless specified otherwise, the term antibody encompasses not only whole antibody molecules, but also antibody fragments as well as variants (including derivatives) of antibodies, such as humanized, chimeric and synthetic antibodies and antibody fragments.

In natural antibodies such as ADAs and ASAs (i.e. mammalian antibodies), two heavy chains are linked to each other by disulfide bonds and each heavy chain is linked to a light chain by a disulfide bond. There are two types of light chain, lambda (1) and kappa (k). There are five main heavy chain classes (or isotypes) which determine the functional activity of an antibody molecule: IgM, IgD, IgG, IgA and IgE. Each chain contains distinct sequence domains. The light chain includes two domains, a variable domain (VL) and a constant domain (CL). The heavy chain includes four domains, a variable domain (VH) and three constant domains (CH1, CH2 and CH3, collectively referred to as CH). The variable regions of both light (VL) and heavy (VH) chains determine binding recognition and specificity to the antigen. The constant region domains of the light (CL) and heavy (CH) chains confer important biological properties such as antibody chain association, trans-placental mobility, complement binding, and binding to Fc receptors (FcR).

The Fv fragment is the N-terminal part of the Fab fragment of an immunoglobulin and consists of the variable portions of one light chain and one heavy chain. The specificity of the antibody resides in the structural complementarity between the antibody combining site and the antigenic determinant. Antibody combining sites are made up of residues that are primarily from the hypervariable or complementarity determining regions (CDRs). Complementarity Determining Regions or CDRs refer to amino acid sequences which together define the binding affinity and specificity of the natural Fv region of a native immunoglobulin binding site. The light and heavy chains of an immunoglobulin each have three CDRs, designated L-CDR1, L-CDR2, L-CDR3 and H-CDR1, H-CDR2, H-CDR3, respectively. An antigen-binding site, therefore, typically includes six CDRs, comprising the CDR set from each of a heavy and a light chain V region. Framework Regions (FRs) refer to amino acid sequences interposed between CDRs. The term *"antibody"* may also include *"single-chain"* (or *"single-domain"*) antibodies, and *"heavy-chain antibodies"* (VHH or VNAR antibodies) also referred herein as *"non-conventional antibodies"* which are found in camelids and sharks.

However, when the term *"antibody"* is considered by reference to the ADAs or ASAs to be detected or quantified, it must necessarily comprise a plurality of antigen-binding sites; accordingly it will be understood herein that those ADAs or ASAs are not single-chain antibodies and/or non-conventional antibodies.

On the other hand, single-chain antibodies and/or non-conventional antibodies, and fragments thereof, may be considered as part of the conjugated drug polypeptide and/or the labeled form of the said drug/polypeptide. For example, the ADAs to be detected or quantified according to the methods of the invention may be directed against conventional therapeutic antibodies, but also non-conventional, humanized, chimeric and/or synthetic therapeutic antibodies.

As used herein, the terms *"variable region"* and *"variable domain"* have the same meaning, and will be used equally in the present invention.

As used herein, the terms *"hypervariable region", "hypervariable domain"* and *"Complementarity Determining Regions (CDRs)"* have the same meaning, and will be used equally in the present invention. Hypervariable regions are necessarily part of variable regions.

As used herein the term *"single domain antibody"* has its general meaning in the art and refers to the single heavy chain variable domain of antibodies of the type that can be found in Camelid mammals which are naturally devoid of light chains. Such single domain antibody are also called VHH or *"nanobody*^{®}*".* For a general description of (single) domain antibodies, reference is also made to the prior art cited above, as well as to EP 0 368 684, Ward et al. (Nature 1989 Oct 12; 341 (6242): 544-6), Holt et al., Trends Biotechnol., 2003, 21(11):484-490; and WO 06/030220, WO 06/003388.

As used herein, a *"human antibody"* is intended to include antibodies having variable and constant regions derived from human immunoglobulin sequences. The human antibodies of the present invention may include amino acid residues not encoded by human immunoglobulin sequences (e.g., mutations introduced by random or site-specific mutagenesis in vitro or by somatic mutation in vivo). However, the term *"human antibody",* as used herein, is not intended to include antibodies in which CDR sequences derived from the germline of another mammalian species, such as a mouse, have been grafted onto human framework sequences.

As used herein, a *"humanized antibody"* refers to an antibody having variable region framework and constant regions from a human antibody but retains the CDRs of a previous non-human antibody.

As used herein, a *"chimeric antibody"* refers to an antibody which comprises a VH domain and a VL domain of a non-human antibody, and a CH domain and a CL domain of a human antibody.

As used herein, a *"synthetic antibody"* refers to an entirely *in vitro* engineered molecule which comprises at least fragments of a variable region of an antibody. Synthetic antibodies, in the sense of the invention, may thus also be antigen-binding fragments selected from the group consisting of a Fab, a F(ab)'2, a single domain antibody (sdAb), a ScFv, a Fab-scFv, a ScFv-Fc, a Sc(Fv)2, a diabody, a di-diabody, a triabody, a tetrabody, a pentabody, an unibody, a minibody, a maxibody, a small modular immunopharmaceutical (SMIP), and fragments which comprise variable regions such as variable light (VL) and variable heavy (HL) chains. Examples of engineered multivalent antibodies which are also considered as antibodies herein are listed in Nuñez-Prado et al. ("The coming of age of engineered multivalent antibodies"; Drug Discovery Today; Vol. 0, N°0; 2015).

The terms *"monoclonal antibody", "monoclonal Ab", "monoclonal antibody composition", "mAb",* or the like, as used herein refer to a preparation of antibody molecules of single molecular composition. A monoclonal antibody composition displays a single binding specificity and affinity for a particular epitope.

The terms *"polyclonal antibody", "polyclonal Ab", "polyclonal antibody composition", "pAb",* or the like, as used herein refer to a preparation of antibody molecules with a plurality of molecular compositions. According to some embodiments, the therapeutic antibodies against which ADAs are directed may thus comprise or consist of polyclonal antibodies or a composition thereof.

As used herein, the term *"specificity"* refers to the ability of an antibody to detectably bind an epitope presented on an antigen while having relatively little detectable reactivity with non-antigen proteins or structures. Specificity can be relatively determined by binding or competitive binding assays, using, e.g., Biacore instruments, a described elsewhere herein. Specificity can be exhibited by, e.g., an about 10:1, about 20:1, about 50:1, about 100:1, 10.000:1 or greater ratio of affinity/avidity in binding to the specific antigen versus nonspecific binding to other irrelevant molecules.

As used herein, the term *"specifically binds to"* or *"binds specifically to"* refers to the ability of an antigen-binding domain (ABD) to bind to an antigen (i.e. an epitope which is present in the conjugated polypeptide and the labeled form of the polypeptide).

As used herein, the term *"affinity",* as used herein, means the strength of the binding of an antibody to an epitope. The affinity of an antibody is given by the dissociation constant Kd, defined as [Ab] x [Ag] / [Ab-Ag], where [Ab-Ag] is the molar concentration of the antibody-antigen complex, [Ab] is the molar concentration of the unbound antibody and [Ag] is the molar concentration of the unbound antigen. The affinity constant Ka is defined by 1/Kd. Preferred methods for determining the affinity of mAbs can be found in Harlow, et al., Antibodies: A Laboratory Manual, Cold Spring Harbor Laboratory Press, Cold Spring Harbor, N.Y., 1988), Coligan et al., eds., Current Protocols in Immunology, Greene Publishing Assoc. and Wiley Interscience, N.Y., (1992, 1993), and Muller, Meth. Enzymol. 92:589-601 (1983), which references are entirely incorporated herein by reference. One method well known in the art for determining the affinity of mAbs is the use of Biacore instruments.

As used herein, the expression *"stable isotope-labeled" or "labeled with a stable isotope"* polypeptide (i.e. an antibody) refers to a polypeptide whose chemical structure (i.e. primary structure), except for the presence of isotope, is identical or substantially identical to the non-labeled polypeptide (i.e. the conjugated drug or polypeptide from the solid support).

As used herein, the *"percentage identity"* between two sequences of proteins means the percentage of identical amino acid residues between the two sequences to be compared, obtained after optimal alignment, this percentage being purely statistical and the differences between the two sequences being distributed randomly along their length. The comparison of sequences is traditionally carried out by comparing the sequences after having optimally aligned them, said comparison being able to be conducted by segment or by using an "alignment window". Optimal alignment of the sequences for comparison can be carried out, in addition to comparison by hand, by means of the local homology algorithm of Smith and Waterman (1981), by means of the local homology algorithm of Neddleman and Wunsch (1970), by means of the similarity search method of Pearson and Lipman (1988) or by means of computer software using these algorithms (GAP, BESTFIT, FASTA and TFASTA in the Wisconsin Genetics Software Package, Genetics Computer Group, 575 Science Dr., Madison, WI, or by the comparison software BLAST NR or BLAST P).

The percentage identity between two sequences is determined by comparing the two optimally-aligned sequences in which the sequence to compare can have additions or deletions compared to the reference sequence for optimal alignment between the two sequences. Percentage identity is calculated by determining the number of positions at which the amino acid residue is identical between the two sequences, preferably between the two complete sequences, dividing the number of identical positions by the total number of positions in the alignment window and multiplying the result by 100 to obtain the percentage identity between the two sequences.

As used herein the expression *"sharing at least 80% sequence identity"* includes sharing at least 80%, 81%, 82%, 83%, 84%, 85%, 86%, 87%, 88%, 89%, 90%, 91%, 92%, 93%, 94%, 95%, 96%, 97%, 98% and 99% identity with a reference polypeptide sequence.

As used herein, the expression *"therapeutic antibodies"* may encompass those selected from a group comprising or consisting of: non-human antibodies, human antibodies, humanized antibodies, synthetic antibodies, chimeric antibodies.

Therapeutic antibodies may be selected from the group consisting of therapeutic antibodies used in inflammation, therapeutic antibodies used in oncology and also immunotherapies. Also, therapeutic antibodies may be selected from the group consisting of : anti-TNF antibodies, anti-VEGF antibodies, anti-EGFR antibodies, anti-PD-1 antibodies, anti-HER2 antibodies, anti-CD20 antibodies, anti-IL17 antibodies, and anti-CTLA4 antibodies, anti-PDLl, anti-CD25, anti-α4integrin, anti-IL6R, anti-C5, anti-IL1, anti-TPO, anti-IL12/23, anti-EPCAM/CD3, anti-CD30, anti-CD80/86, anti-anthrax, anti-CCR4, anti-CD6, anti-CD19, anti-α4β7, anti-IL6, anti-VEGFR-2, anti-SLAMF7, anti-GD2, anti-IL17A, anti-PCSK9, anti-IL5, anti-CD22, anti-IL4, anti-PDGFRa, anti-IL17RA and anti-TcdB.

Illustratively, reference therapeutic antibodies or fusion proteins or therapeutic proteins may be selected in a group consisting of: 3F8, Abagovomab, Abatacept, Abciximab, Abituzumab, Abrezekimab, Abrilumab, Actoxumab, Adalimumab, Adecatumumab, Ado-trastuzumab emtansine, Aducanumab, Aflibercept, Afutuzymab, Afasevikumab, Afelimomab, Alacizumab, Alefacept pegol, Alemtuzumab, Alirocumab, Altumomab, Amatixumab pentetate, Amatuximab, Amivantamab, Anatumomab mafenatox, Andecaliximab, Anetumab, Anifromumab ravtansine, Anifrolumab, Ansuvimab, Anrukinzumab, Apolizumab, Aprutumab ixadotin, Arcitumomab, Ascrinvacumab, Aselizumab, Atezolizumab, Atidortoxumab, Atinumab, Altizumab, Atoltivimab, Atorolimumab, Avelumab, Azintuxizumab vedotin, Bamlanivimab, Bapineuzumab, Basiliximab, Bavituximab, BCD-100, Bectumomab, Begelomab, Belatacept, Belantamab mafodotin, Belimumab, Bemarituzumab, Benralizumab, Berlimatoxumab, Bermekimab, Bersanlimab, Bertilimumab, Besilesomab, Bevacizumab, Bezlotoxumab, Biciromab, Bimagrumab, Bimekizumab, Birtamimab, Bivatuzumab, Bleselumab, Blinatumomab, Blontuvetmab, Blosozumab, Bococizumab, Brazikumab, Brentuximab vedotin, Briakinumab, Brodalumab, Brolucizumab, Brontictuzumab, Burosumab, Cabiralizumab, Camidanlumab tesirine, Camrelizumab, Canakinumab, Cantuzumab mertansine, Cantuzumab ravtansine, Caplacizumab, Casirivimab, Capromab, Carlumab, Carotuximab, Catumaxomab, cBR96-doxorubicin immunoconjugate, Cedelizumab, Cemiplimab, Cergutuzumab amunaleukin, Certolizumab pegol, Cetuximab, Cetrelimab, Cetuximab, Cibisatamab, Cirmtuzumab, Citatuzumab bogatox, Cixutumumab, Clazakizumab, Clenoliximab, Clivatuzumab tetraxetan, Codrituzumab, Cofetuzumab pelidotin, Coltuximab ravtansine, Conatumumab, Concizumab, Cosfroviximab, Crenezumab, Crizanlizumab, Crotedumab, CR6261, Cusatuzumab, Dacetuzumab, Daclizumab, Dalotuzumab, Dapirolizumab pegol, Daratumumab, Dectrekumab, Demcizumab, Denintuzumab mafodotin, Denosumab, Depatuxizumab mafodotin, Derlotuximab biotin, Detumomab, Dezamizumab, Dinutuximab, Dinutuximab beta, Diridavumab, Domagrozumab, Dorlimomab aritox, Dostarlimab, Drozitumab, DS-8201, Duligotuzumab, Dupilumab, Durvalumab, Dusigitumab, Duvortuxizumab, Ecromeximab, Eculizumab, Edobacomab, Edrecolomab, Efalizumab, Efungumab, Eldelumab, Elezanumab, Elgemtumab, Elotuzumab, Elsilimomab, Emactuzumab, Emapalumab, Emibetuzumab, Emicizumab, Enapotamab vedotin, Enavatuzumab, Enfortumab vedotin, Enlimomab pegol, Enoblituzumab, Enokizumab, Enoticumab, Ensituximab, Epcoritamab, Epitumomab cituxetan, Epratuzumab, Eptinezumab, Erenumab, Erlizumab, Ertumaxomab, Etanercept, Etaracizumab, Etesevimab, Etigilimab, Etrolizumab, Evinacumab, Evolocumab, Exbivirumab, [fam-]trastuzumab deruxtecan, Fanolesomab, Faralimomab, Faricimab, Farletuzumab, Fasinumab, FBTA05, Felvizumab, Fezakinumab, Fibatuzumab, Ficlatuzumab, Figitumumab, Firivumab, Flanvotumab, Fletikumab, Flotetuzumab, Fontolizumab, Foralumab, Foravirumab, Fremanezumab, Fresolimumab, Frovocimab, Frunevetmab, Fulranumab, Futuximab, Galcanezumab, Galiximab, Gancotamab, Ganitumab, Gantenerumab, Gatipotuzumab, Gavilimomab, Gedivumab, Gemtuzumab ozogamicin, Gevokizumab, Gilvetmab, Gimsilumab, Girentuximab, Glembatumumab vedotin, Golimumab, Gomiliximab, Gosuranemab, Guselkumab, Ianalumab, Ibalizumab, Sintilimab, Ibritumomab tiuxetan, Icrucumab, Idarucizumab, Ifabotuzumab, Igovomab, Iladatuzumab vedotin, Imalumab, Imaprelimab, Imciromab, Imdevimab, Imgatuzumab, Inclacumab, Indatuximab ravtansine, Indusatumab vedotin, Inebilizumab, Infliximab, Intetumumab, Inolimomab, Inotuzumab ozogamicin, Ipilimumab, Iomab-B, Iratumumab, Isatuximab, Iscalimab, Istiratumab, Itolizumab, Ixekizumab, Keliximab, Labetuzumab, Lacnotuzumab, Ladiratuzumab vedotin, Lampalizumab, Lanadelumab, Landogrozumab, Laprituximab emtansine, Larcaviximab, Lebrikizumab, Lecanemab, Lemalesomab, Lendalizumab, Lenvervimab, Lenzilumab, Lerdelimumab, Leronlimab, Lesofavumab, Letolizumab, Lexatumumab, Libivirumab, Lifastuzumab vedotin, Ligelizumab, Loncastuximab tesirine, Losatuxizumab vedotin, Lilotomab satetraxetan, Lintuzumab, Lirilumab, Lodelcizumab, Lokivetmab, Lorvotuzumab mertansine, Lucatumumab, Lulizumab pegol, Lumiliximab, Lumretuzumab, Lupartumab, Lupartumab amadotin, Lutikizumab, Maftivimab, Mapatumumab, Margetuximab, Marstacimab, Maslimomab, Mavrilimumab, Matuzumab, Mepolizumab, Metelimumab, Milatuzumab, Minretumomab, Mirikizumab, Mirvetuximab soravtansine, Mitumomab, Modotuximab, Mogamulizumab, Monalizumab, Morolimumab, Mosunetuzumab, Motavizumab, Moxetumomab pasudotox, Muromonab-CD3, Nacolomab tafenatox, Namilumab, Naptumomab estafenatox, Naratuximab emtansine, Narnatumab, Narsoplimab, Natalizumab, Navicixizumab, Navivumab, Naxitamab, Nebacumab, Necitumumab, Nemolizumab, NEOD001, Nerelimomab, Nesvacumab,Netakimab, Nimotuzumab, Nirsevimab, Nivolumab, Nofetumomab merpentan, Obiltoxaximab, Obinutuzumab, Ocaratuzumab, Ocrelizumab, Odesivimab, Odulimomab, Ofatumumab, Olaratumab, Oleclumab, Olendalizumab, Olokizumab, Omalizumab, Omburtamab, OMS721, Onartuzumab, Ontuxizumab, Onvatilimab, Opicinumab, Oportuzumab monatox, Oregovomab, Orticumab, Otelixizumab, Otilimab, Otlertuzumab, Oxelumab, Ozanezumab, Ozoralizumab, Pagibaximab, Palivizumab, Pamrevlumab, Panitumumab, Pankomab, Panobacumab, Parsatuzumab, Pascolizumab, Pasotuxizumab, Pateclizumab, Patritumab, PDR001, Pembrolizumab, Pemtumomab, Penpulimab, Perakizumab, Pertuzumab, Pexelizumab, Pidilizumab, Pinatuzumab vedotin, Pintumomab, Placulumab, Prezalumab, Plozalizumab, Pogalizumab, Polatuzumab vedotin, Ponezumab, Porgaviximab, Prasinezumab, Prezalizumab, Priliximab, Pritoxaximab, Pritumumab, PRO 140, Quilizumab, Racotumomab, Radretumab, Rafivirumab, Ralpancizumab, Ramucirumab, Ranevetmab, Ranibizumab, Raxibacumab, Ravagalimab, Ravulizumab, Refanezumab, Regavirumab, Regdanvimab, Relatlimab, Remtolumab, Reslizumab, Retifanlimab, Rilonacept, Rilotumumab, Rinucumab, Risankizumab, Rituximab, Rivabazumab pegol, Robatumumab, Rmab, Roledumab, Romilkimab, Romosozumab, Rontalizumab, Rosmantuzumab, Rovalpituzumab tesirine, Rovelizumab, Rozanolixizumab, Ruplizumab, SA237, Sacituzumab govitecan, Samalizumab, Samrotamab vedotin, Sarilumab, Satralizumab, Satumomab pendetide, Secukinumab, Selicrelumab, Seribantumab, Setoxaximab, Setrusumab, Sevirumab, Sibrotuzumab, SGN-CD19A, SHP647, Sifalimumab, Siltuximab, Simtuzumab, Siplizumab, Sirtratumab vedotin, Sirukumab, Sofituzumab vedotin, Solanezumab, Solitomab, Sonepcizumab, Sontuzumab, Sotrovimab, Spartalizumab, Spesolimab, Stamulumab, Sulesomab, Suptavumab, Sutimlimab, Suvizumab, Suvratoxumab, Tabalumab, Tacatuzumab tetraxetan, Tadocizumab, Tafasitamab, Talacotuzumab, Talizumab, Talquetamab, Tamtuvetmab, Tanezumab, Taplitumomab paptox, Tarextumab, Tavolimab, Tebentafusp, Teclistamab, Tefibazumab, Telimomab aritox, Telisotuzumab, Telisotuzumab vedotin, Tenatumomab, Teneliximab, Teplizumab, Tepoditamab, Teprotumumab, Tesidolumab, TGN 1412, Tetulomab, Tezepelumab, TGN1412, Tibulizumab, Tildrakizumab, Tigatuzumab, Timigutuzumab, Timolumab, tiragolumab, Tiragotumab, Tislelizumab, Tisotumab vedotin, TNX-650, Tocilizumab, Tomuzotuximab, Toralizumab, Toripalimab, Tosatoxumab, Tositumomab, Tovetumab, Tralokinumab, Trastuzumab, Trastuzumab duocarmazine, Trastuzumab emtansine, TRBS07, Tregalizumab, Tremelimumab, Trevogrumab, Tucotuzumab celmoleukin, Tuvirumab, Ublituximab, Ulocuplumab, Urelumab, Urtoxazumab, Ustekinumab, Utomilumab, Vadastuximab talirine, Vanalimab, Vandortuzumab vedotin, Vantictumab, Vanucizumab, Vapaliximab, Varisacumab, Varlilumab, Vatelizumab, Vedolizumab, Veltuzumab, Vepalimomab, Vesencumab, Visilizumab, Vobarilizumab, Volociximab, Vonlerolizumab, Vopratelimab, Vorsetuzumab mafodotin, Votumumab, Vunakizumab, Xentuzumab, XMAB-5574, Zalutumumab, Zanolimumab, Zatuximab, Zenocutuzumab, Ziralimumab, Zolbetuximab, Zolimomab aritox.

As used herein the expression *"Anti-Self Antibody"* or "*ASA"* or *"auto-antibody"* refers to any naturally occuring antibody produced by the immune system that is directed against one or more of the individual's own proteins. Unless instructed otherwise, the term may thus include any auto-antibody, including those which may (or may not) be associated with a specific pathological condition. An *"autoantibody"* binds to an analyte that occurs in the individual in which the antibody is produced because the individual's immune system recognizes the analyte (typically a protein or polypeptide) as foreign even though that antigen actually originated in the individual. Generally the analyte is one that occurs naturally in a subject, including analytes that are the result of or arise from a disease or disease process (e.g., altered forms of naturally occurring proteins produced by a diseased cell or during a disease process). For example, an autoantibody to an endogenous cancer-correlated antigen is an autoantibody produced by the subject's immune system that binds an endogenous antigen correlated with occurrence of disease, e.g., cancer.

In particular, examples of ASAs which can be detected and/or quantified by the methods described herein include: Anti-centromere antibodies, Anti-dsDNA, Anti-gp210, Anti-histone antibodies, Anti-Jo1, Anti-La/SS-B, Anti-p62, Anti- Polymyositis/systemic sclerosis (PM-Scl), Anti-RNP, Anti-Ro/SS-A, Anti-Sm (Smith), Anti-splOO, Anti-topoisomerase, Anti-CCP, Anti-ganglioside antibodies, Anti-mitochondrial antibody, Antiphospholipid antibodies, Anti-smooth muscle antibody, Anti-SRP, Anti-thyroid autoantibodies, Anti-transglutaminases, c-ANCAs, anti-Liver kidney microsomal type 1, Lupus anticoagulant, p-ANCAs, Rheumatoid factor, anti-sperm antigens.

In particular, examples of ASAs which are commonly reported in the most common types of cancer include: Anti-DNA, Anti-Sm, Anti-p53, Anti-Ro (SSA), Anti-La (SSB), Anti-phospholipid, Anti-c-myc, Anti-HSP60, ANA, Anti-GAD65, Anti-HSP90, Anti-RNP, Anti-alpha-enolase, Anti-Collagen I, Anti-Collagen III, Anti-Collagen V, ANCA, Anti-centromere, Anti-tyrosinase.

Examples of ASAs which are commonly reported in autoantibody mediated tissue damage include: Anti-red blood cells, Anti-platelets, Anti-acetylcholine recepto, Anti-desmoglein 3, Anti-TSH, Anti-intrinsic factor, Anti-IgG, Anti-Ro, Anti-La.

Examples of ASAs which are commonly reported in rheumatic diseases include: Anti-ARS (Aminoacyl-tRNA synthetase), anti-Jo-1 (histidyl-tRNA synthetase), anti-PL-7 (threonyl-tRNA synthetase), anti-PL-12 (alanyl-tRNA synthetase), anti-OJ (isoleucyl-tRNA synthetase), anti-EJ(glycyl-tRNA synthetase), anti-KS (asparaginyl-tRNA synthetase), anti-Ha (tyrosyl-tRNA synthetase), anti-Zo (phenylalanyl-tRNA synthetase), Anti-SRP (Signal recognition particle), Anti-Mi2 (Nucleosome remodeling deacetylase), Anti-MDA5 (Melanoma differentiation-associated protein 5), Anti-Mi2 (Nucleosome remodeling deacetylase), Anti-TIFl (Transcriptional intermediary factor 1), Anti-NXP2 (Nuclear matrix protein 2), Anti-HMGCR (3-hydroxy-3-methylglutaryl-coenzyme A reductase), Anti-SAE (SUMO activating enzyme subunit 1).

Examples of ASAs which are commonly reported in scleroderma include: Anti-centromere (Centromeric protein), Anti-topoisomerase I (Topoisomerase I), Anti-RNA polymerase (RNA polymerase), Anti-U3 RNP (Fibrillarin complexed with small nucleolar RNA U3), Anti-Th/To (Human Rnase MRP complex), Anti-U11/U12 RNP (Small nucleolar RNA U11/U12), Anti-PDGFR (Platelet-derived growth factor receptor), Anti-M3R (Type 3 muscarinic receptor), Anti-ATIR (Angiotensin II type 1 receptor), Anti-ETAR(Endothelin-1 type A receptor).
- Illustratively, and in a non-exhaustive manner, immunogenic proteins/polypeptides against which ASAs have been identified (i.e. immunogenic proteins capable of binding to the said ASAs) and which may further be conjugated to a solid support acording to the present disclosure include: ATG16L1, ABCF2, ABL2, TNK2, EIF2C4, APOL2, APRT, AQP2, ARL6IP4, ARHGEF1, ARHGEF16, ARIH2, ARMC1, ARMC6, ASCC2, ASF1B, ASPH, ASPHD1, ASS1, ATF1, ATG4A, IGK, AZI2,BCL2L12, BCL2L14, HSPC142, C17orf49, BCL11A, BCCIP, BCKDK, BID,
   BNIP3L, DC42EP4, C7orf27, BYSL, BZW2, C1orf105, C1orf131, CHAF1B, CARD9, CBS, CCDC97, CDC123, CDCA3, CDK6, CENPB, CENPQ, CEP57, VPS24, CHRAC1, C1orf77, C9orf142, CINP, CKAP2, COASY, COG3, COPZ1, CIAPIN1, RCSD1, C18orf25, CRK, CSF1R, CSNK2A1, GPS1, CTDSPL, CTNNAL1, PSCD4, DYNC1LI2, AMD1, GAD2, DCLK1, DDX19B, DDX21, DDX54, DEF6, DGCR8, DHX8, DMD, DNAJC8, LIG3, C19orf26, DPM1, POLE, DPYSL3, DPYSL4, DPYSL5, DTNBP1, DTL, DDX39A, DYNC1H1, ECE1, EIF3S4, ELOVL5, EME1, C11orf30, EPM2AIP1, ETS1, ETS2, FAM125A, MGC40405, C14orf24, C9orf25, FAM63B, FKBP1A, WTAP, FMNL2, FMR1, FKSG44, FYN, GPI, TUBGCP3, SIP1, GGA1,GSK3B, HIST2H2BE, HIST3H2BB, UCHL5IP, HCK, HDAC9, HMGB1, HMGB2, HNRNPK, HSF1, EIF4H, IGF1R, IMPDH2, IFNGR2, ITM2C, JAK1, JAK3, C22orf9, C12orf41, PRKAR2B, CSNK1G3, KDM2A, JMJD2D, KLC2, KLC3, KLHDC4, KLF3, RPS6KA6, RPS6KB2, LARP2, LARP1, LARP4, LARP7, LUC7L2, LCK, LRBA, LRRFIP1, MAP3K2, MAP4K4, MAN2C1, MAGEB2, MAPKAPK2, MARK1, MAX, MBIP, MCF2L, MCM2, MCM6, MED1, MED4, C2orf33, FLJ20323, MAPK6, MAPK13, MKNK2, MAP2K3, CDC42BPA, MSL3L1, MST4, CBFA2T2, MTMR2, MTMR14, MYLK, NADK, NCF1, NFIC, ZCCHC17, NOL7, HSPC111, NPM1, NUP133, NUDT9, OSBPL11, OSBPL1A, PIK3C2A, PCYT1B, PDCD4, PELO, PGM1, PGM2, PLEKHA3, LCP1, PNPT1, PPP1R14A, PSMC4, PSIP1, PSMD4, PSRC1, PTEN, PTPN18, RAB3IP, RAB9A, RASSF3, hCG_20114, RBM39, RBM4B, RAB11FIP2, RING1, RIOK1, RPAIN, RPL12, RSL1D1, RPL30, RPL31, RNF138, ELAC2, ROCK1, POLR2I, POLR3C, RSRC2, MRPS18B, SNRPA1, RUSC1, RUVBL2, SLC23A2, SAE2, SAMHD1, CIP29, SCLT1, SCML1, SELPLG, SENP2, SEPP1, SF3B2, SH2B1, SIN3A, SKIL, SMARCA5, RY1, USP39, SNX1, SP100, SPAG6, SPC24, CCDC99, APEG1, SPHK2, SQSTM1, SFRS6, SSH3, STK33, STK4, STRAP, TSC22D3, GTF2F1, MAP3K7IP1, TAGLN2, TALDO1, TBC1D22A, TBCB, TCF19, SUB1, CCT8, TNS1, TGFBR2, TTN, TPD52L2, TPI1, TRDN, TRPT1, TRPV2, TSGA10, TTC1, TEX264, TXNL4A, TYK2, TYMS, UBE2O, USP15, UCK2, VDP, VAMP4, VAPB, VPS37A, WWC2, WWP2, YES1, ZAP70, LOC730411, CCDC131, ZC3H3, ZHX1, ZNF174, ZNF326, ZRANB2, ANPEP, CCDC90B, DDX43, DPP3, ERR beta-LBD, EXOC5, FTH1, GAGE1, IL10RB, KCNIP2, LSM12, MAP3K14, MAPK14, RUNX1T1, MGC42105, NRBP1, PHYHD1, PLSCR1, RNF7, SGK2, TBN, TAF9B, TPM3, TTYH2, OGN, PIR, FNTB, SERPINB3, LSM3, CSE1L, CSTB, IDH1, TRUB1, ATIC, RFX5, METTL9, S100A7A, SUMO2, PTBP2, ALDOB, GAGE1, CRKL, UBQLN1, EIF1, COQ3, PPP2R1B, GSDML, IL1F8, CTBP2, UBE2V1, GAD1, NUDCD1, UNC119, YKT6, ODF3L2, RWDD4A, GGA3, HPRT1, TCEAL5, SKP1A, ZADH1, AHSA1, PSME2, AGPAT1, C5orf15, ZBED1, C17orf62, UBE2S, RSU1, RAB3IL1, SDF4, ANXA5, PABPC5, SORD, PSMD10, C3orf26, NCF2, UCK1, PITPNB, WARS, DUSP12, COG8, LAIR1, CASP3, AKR1B10, COQ9, HOXA1, THOP1, GSN, UBA3, GABRA6, SLC22A7, RRM1, EIF2C1, GCC1, USP4, FAM13A1, PFKL, SF3B3, GMFB, C7orf16, MOK, FGF2, aa1-155, SERPINA3, APTX, ARNTL, C20orf43, CYFIP2, CYGB, EIF2B1, FLOT2, GIMAP5, GMDS, HK1, MAGEA4, MAGEA10, MLX, MPP3, NACA2, CYB5R4, NECAP2, PANK1, PDE9A, PIGG, PITPNA, PLCD1, POU5F1, PTMS, DSCR1, RPL22, MRPS27, SAP18, DNTT, TGM4, VSNL1, VPS26A, RCHY1, ZSCAN21, and/or
- Ro-RNP complex, La antigen, Small nuclear RNP, Histones, Topoisomerase I, Centromere, Myeloperoxidase (p-ANCA), proteinase 3 (c-ANCA), Cardiolipin, Citrullinated protein, Carbamylated protein, Rheumatoid Factor (RF), Collagen, Type IV collagen, and/or

SSA, SSB, Ro52, Ro60, La, RNA-binding proteins, RNP-A, U1-70K, Sm-D3, Jo1, PL7, threonyl t-RNA synthetase, PL-12 alanyl-tRNA synthetase, Mi2 histone acetylase, tRNA synthetase proteins, TIF-γ, MDA-5, 3-hydroxy-3-methylglutaryl-coenzyme A reductase, centromere proteins, CENPA, CENPB, PM/SCL, EXOSC9, EXOS107, RNA polymerase 3 complex, POLR3A, POLR3K, insulin, IA-2, glutamate decarboxylase/GAD65, Znt8, tetraspanin-7, peptidylpropyl isomerase like 2, Mlhl, proteinase-3, myeloperoxidase, nicotinic acetylcholine receptor/nAChR, muscle-associated cell surface tyrosine kinase, low-density lipoprotein receptor-related protein, aquaporin-4, myelin oligodendrocyte glycoprotein, N-methyl-D-asparate receptor/NMDAR, alpha-amino-3-hydroxy-5methyl-4-isoxazolepropionic receptor/AMPAR, leucine rich glioma inactivated 1 protein, neurexin family protein contactin associated protein 2, phospholipase A2 receptor, thrombospondin type-1 domain-containing 7A, GMCSF, interferon-y, CHRNA1, LRP4, GPIHBP1, GluRl, ADAMTS13, FGF23, CSF2RA, IFNGR, PLA2R; and fragments thereof.

As used herein, the expression *"solid support"* applies indifferently to any solid support which may comprise any suitable solid carrier such as, for example, particulate materials, porous members possessing an intercommunicating network of openings throughout such that a fluid deposited on the member will propagate through the member, and the like. Suitable porous members include porous membranes, fibrous mesh materials and the like and may be of any suitable material such as glass, polymeric materials, paper, etc, as long as the provided solid support is capable of being conjugated to a polypeptide or a polypeptide-based drug, such as a therapeutic antibody, or any other polypeptide such as a recombinant form of an immunogenic polypeptide (i.e. a peptide which is prone to induce and/or bind specifically to an anti-self antibody). In a non-exhaustive manner, a solid support may thus comprise, or consist of particles (including microparticles and beads) made from materials such as polymer, metal (paramagnetic, ferromagnetic particles), glass, and ceramic; gel substances such as silica, alumina, and polymer gels; capillaries, which may be made of polymer, metal, glass, and/or ceramic; zeolites and other porous substances; electrodes; microtiter plates; solid strips; and cuvettes, tubes or other spectrometer sample containers.

A solid support component of an assay is distinguished from inert solid surfaces with which the assay may be in contact in that a *"solid support"* contains at least one moiety on its surface, which is intended to interact with the drug polypeptide (e.g. the therapeutic antibody, immunogenic protein, or fragment thereof). A solid phase may be a stationary component, such as a tube, strip, cuvette or microtiter plate, or may be non-stationary components, such as beads and microparticles. Microparticles can also be used as a solid phase for homogeneous assay formats. A variety of microparticles that allow either non-covalent or covalent attachment of proteins and other substances may be used. Such particles include polymer particles such as polystyrene and poly(methylmethacrylate); gold particles such as gold nanoparticles and gold colloids; and ceramic particles such as silica, glass, and metal oxide particles.

As used herein the expression *"solid phase"* is meant to refer to the part of a reaction which includes the solid support, and any other compound which is retrievable with said solid support during Solid Phase Extraction. The part of the assay which is not part of the solid phase is referreing herein as the *"mobile phase".*

As used herein, the expression *"calibration curve",* or *"titration curve",* is meant to refer to any data reporting a measured signal or amount (e. g. an absolute or relative concentration) of one or more analyte(s), as a function of a corresponding expected signal or amount of one or more analyte(s), which may be the same analyte(s) or different analyte(s).

Accordingly, and in a non-exhaustive manner, this expression may encompass a calibration curve reporting a measured signal or amount of one or more proteotypic peptides as a function of a corresponding expected signal or amount of one or more ADAs, ASAs, or mixtures thereof.

As used herein, the expression *"calibrator"* is meant to refer to any reagent and/or compound for generating a calibration curve. For example, a calibrator may comprise or consist of an antibody. In particular, the expression *"calibrator"* may refer to any polypeptide or polypeptide-based construct corresponding to, or mimicking, an ADA or ASA susceptible to be detected or quantified in a sample, such as an anti-Infliximab ADA, an anti-Adalimumab ADA and mixtures thereof.

In a non-exhaustive manner, a calibrator may thus refer to ADAs, ASAs, and mixtures thereof, diluted in a blank matrix (e.g. human plasma) at different concentrations; for example over a concentration range from about 0 ng/mL to about 1 µg/mL, which includes about 0, 10, 20, 50, 100, 200, 350, 500 ng/mL, 1 µg/mL, or any other sub-range thereof.

The embodiments of the invention will be further defined here below.

### Methods for detecting and/or quantifying ADAs and ASAs

As it is readily understood from the present specification, the detection and quantification methods described herein are particularly useful (i) in situations wherein a tested patient has received a therapeutic treatment by administration of one or more therapeutic drugs, in particular one or more therapeutic antibodies and (ii) in situations wherein a tested patient is suspected to have developed antibodies against the said drug (ADAs) and/or (iii) in situations wherein a tested patient is suspected to have developed auto-antibodies (ASAs).

As illustrated in the examples herein, the inventors have shown that a precise detection of antibodies (i.e. ADAs and ASAs) in a sample (e.g. a human sample) may be performed through the design of a method wherein the antibodies, if present in the said sample, are determined by a mass spectrometry method making use of proteolysis peptide(s) derived from the labeled form or the polypeptide(s) recovered, or recoverable, from a the solid support, which may thus comprise the step of:
(A) determining the mass spectrometry signal generated by one or more selected labeled proteotypic peptides from the labeled forms of the polypeptides, and
(B) determining the ADAs and/or ASAs, if present, in the said sample based on the signal at step (A).

As illustrated in the examples herein, the inventors have shown that a precise quantification of antibodies (i.e. ADAs and ASAs) in a sample (e.g. a human sample) may further be performed and improved through the design of a method wherein the amount of antibodies, if present in the said sample, is determined by a mass spectrometry method making use of proteolysis peptide(s) derived from the labeled form or the polypeptide(s) recovered, or recoverable, from a the solid support, which may thus comprise the step of :
(A) calculating a ratio between:
   (i) the mass spectrometry signal generated by one or more selected labeled proteotypic peptides from the labeled forms of the polypeptides, and
   (ii) the mass spectrometry signal generated by one or more of the corresponding unlabeled proteotypic peptides from the proteolysis of the polypeptide; conjugated to the solid support.
(B) determining the amount of ADAs and/or ASAs, if present, in the said sample by reporting the ratio value calculated at step (A) for each of the one or more proteolysis peptide to a calibration curve of ratio values (e.g. acquired from the calibrators processed with the same process); thereby quantifying the said antibodies.

Depending on the experimental setting, the peptides which are susceptible to be generated following the step of enzyme proteolysis, and thus susceptible to be present in the proteolysis sample, may include:
- peptides from the ADAs or ASAs which are to be detected or quantified in the sample or peptides from reference ADAs and ASAs (e.g. commercial ADAs or ASAs) which may serve for calibration.
- labeled proteotypic peptides (SIL) which are used to indirectly detect and quantify ADAs or ASAs;
- peptides from the previously conjugated drug or polypeptide and which may serve for normalization (IS).

Examples of quantification are provided in **figure 3** of the present specification.

### Selecting proteotypic peptides

Proteotypic peptides derived from a given reference to be monitored by mass spectrometry when performing the detection/quantification method described herein may be selected according to selection methods that are known from the one skilled in the art.

In the context of a method for detecting or quantifying ADAs or ASAs, the proteotypic peptides of interest are obtained from the polypeptide/ligand against which the ADAs and ASAs are directed to (e.g. the immunogenic peptide).

Indeed, proteotypic peptides from the ADAs and ASAs are not necessarily known and the detection or quantification is thus indirect throughout the determination of labeled proteotypic peptides (SIL).

When the present ADA or ASA detection and/or quantification method is performed by starting with a sample, in particular a biological sample such as a human sample, and especially a human plasma sample or a human serum sample, the proteolysis peptides shall be selected so as to be discriminant with regards to proteolysis peptides susceptible to be generated by the action of a protease, e.g. trypsin or IdeS, on other endogenous or exogenous polypeptides and/or antibodies (e.g. in the context of ADAs directed against therapeutic antibodies) that are susceptible to be present in the said sample, e.g. the said human plasma sample or the said human serum sample.

Usually, *'signature'* peptides, which are peptides unique for the specific target protein, are chosen as the surrogate peptides. Selection of the appropriate surrogate peptides should: retain peptides with appropriate length (~ 8-20 amino acids): being too short may cause the lack of selectivity, and too long may affect the sensitivity (Wu et al., Rapid Commun Mass Spectrom. 2011; Vol. 25:281-90).

A typical procedure is to perform an *in silico* digestion of a given reference polypeptide to generate a set of potential surrogate peptides. These peptides are then searched against all existing proteins in the biological matrices using online databases (e.g., Standard Protein BLAST, http://www.ncbi.nlm.nih.gov/BLAST/Blast.cgi?PAGE =Proteins) to confirm that the signature peptides only exist in the target protein. The sensitivity, specificity and chromatographic behavior of these signature peptides are then evaluated using actual digested protein samples in biological matrices, and the best one(s) will be chosen as the surrogate peptide(s) for the said given polypeptide/antibody.

The proteotypic peptides may be selected based on online *in silico* prediction tools (Kamiie et al., Pharmaceutical Research, vol. 25(6): 1469-1483, 2008).

As used herein, proteotypic peptides, which may also be termed surrogate peptides herein, are selected on the basis on their uniqueness among the peptides that may be present after subjecting a given sample (e.g. a human plasma or human serum) to a protease. Accordingly, each selected proteotypic peptide consists of a unique signature of the presence of a given polypeptide in a sample.

It will be understood that it is the SIL-peptides which provide evidence of the presence and/or amount of the corresponding ADA or ASA in the sample; hence, and particularly in the context of a detection or quantification of ADAs, those proteotypic peptides which correspond to fragments of the drug polypeptide are theorically not found endogenously.

As a consequence, at least in the context of kits and methods for detecting or quantifying ADAs, any SIL-peptide from the drug polypeptide is potentially a suitable proteotypic peptide.

For a given ADA or ASA to be detected or quantified with the method described herein, the selection of one or more proteotypic peptide(s) (i.e. "surrogate peptide(s)") may be performed by comparing: (i) a set of the expected proteotypic peptides derived from the said given polypeptide susceptible to bind to the said antibody with (ii) a set of the proteotypic peptides that are expected to be derived from the same proteolysis of proteins, in particular of human plasma or human serum proteins, or even a set of the proteotypic peptides that are expected to be derived from the same proteolysis of ADAs and ASAs.

In some embodiments, the set of expected proteotypic peptides may be obtained *in silico* by using the query *peptide mass* on the online bioinformatics tool www.expasy.ch after entering on the tool (i) the sequence of a given antibody and (ii) the sequences of the proteins that are expected to be contained in the sample, such as the sequences of therapeutic antibodies, such as human IgG.

Then, peptides found exclusively in the set of proteotypic peptides derived from the said given reference polypeptide susceptible to bind to the said antibodies and which are thus preferably not found in the set of proteotypic peptides derived from proteins that are expected to be contained in the sample are selected.

The selection of proteotypic peptides derived from the said given polypeptide may also be performed *in silico,* by performing a similarity research by sequence alignment against a human protein database such as the UniProtKB_HUMAN database, and by using a relevant bioinformatics software, e.g. the bioinformatics tool termed BLAST 2.0 (*Basic Local Alignment Search Tool*)*.* Selection of the one or more proteolysis peptide(s) for LC-MS/MS quantification shall generally take into account of the specificity of the selected proteotypic peptide.

Among the set of one or more potential proteotypic peptides pre-selected as described above, those potential peptides with missed cleavage sites by the protease are excluded. Missed cleavage sites may be predicted by using the software called MC:pred (Lawless et al., OMICS, Sep 2012, Vol. 16(9)).

Alternative methods for quantifying antibodies in a sample of an individual based on the signal emanating from labeled proteolysis peptides can be, for instance, as described in EP3371602B1 or EP3165922B1**.**

The detection and quantification method that is described herein allows the quantification of two or more ADAs and ASAs, and in a multiplex manner, irrespective of the identity of the said antibodies. The antibodies to be quantified by the method described herein may be any antibodies (i.e. ADAs and/or ASAs or a mixture thereof) with specificity for a given drug or self-antigen (e.g. immunogenic protein), such as any ADA directed against a therapeutic antibody e.g. any therapeutic antibody that is the subject of a marketing authorization, at the time of performing the said antibody detection & quantification method, and any ASA directed against a given antigen.

### Generating a calibration curve

The precise detection and quantification of antibodies by mass spectrometry analysis is allowed by the determination of the signal for proteotypic labeled peptides from the labeled polypeptide recovered with the solid phase.

According to some embodiments, a multiplex detection and quantification of a plurality of ADAs or ASAs by mass spectrometry analysis may be achieved by the determination of the signal for a plurality of proteotypic labeled peptides from a plurality of labeled polypeptide recovered with the solid phase, namely at least one labeled polypeptide for each polypeptide against which ADAs/ASAs are directed.

As further detailed in the present specification, a mass spectrometry signal value may consist of the peak area of specific SRM (Selected Reaction Monitoring), or more precisely of the sum of the peak areas of specific SRM transitions, monitored for a selected peptide of interest, typically a selected surrogate tryptic peptide derived from the selected labeled polypeptide (SIL-polypeptide) described herein.

A calibration curve may then be generated by plotting the serial or set of calculated ratio values versus the corresponding theoretical amounts of the selected ADA or ASA (e.g. commercial ADA or ASA). In this case the ratio values correspond to the ratio between the peak area generated by the SIL-polypeptide and the peak area generated by the internal standard (IS).

In the context of methods for detecting and quantifying ADAs or ASAs according to the present disclosure, the internal standard (IS) may correspond to the fraction of the polypeptide released from the solid support (e.g. the therapeutic antibody or immunogenic polypeptide). See for example **figure 3** which illustrate the quantification of ADAs from a sample.

As it will be further detailed in the present specification, the quantification of antibodies is advantageously, performed by reporting the ratio value calculated for each proteotypic peptide considered in the proteolyzed sample tested, or test sample, to a calibration curve of ratio values generated, for each polypeptide, with known amounts of the said reference.

According to some embodiments, the reference may comprise or consist of commercial ADAs, ASAs, mixtures thereof and fragments thereof.

In a non-exhaustive manner, for generating a calibration curve, a serial or set of calibration samples (CS) may be prepared, wherein the serial or set of calibration samples are prepared so as to cover an amount range encompassing at least the amount range which can be recovered after binding to the antibody(ies) (i.e. ADAs or ASAs) which is(are) expected to be contained in a test sample and under a given set of conditions.

Thus, according to the antibody quantification method described herein, a calibration curve may be generated for each polypeptide of interest which is relevant to the detected or quantified ADAs, ASAs, or mixtures thereof. In other embodiments, a calibration curve may be generated simultaneously for a plurality of polypeptides.

For example, the amount of ADAs that may be found in a test sample, especially in a test sample consisting of a human plasma sample originating from a patient treated by therapeutic antibodies, may vary, depending of (i) the amount of therapeutic antibody(ies) which has(have) been administered to the said patient, (ii) the time period when the plasma sample has been collected since the starting time period of the treatment, (ii) the time period of collection of the plasma sample since the last administration of therapeutic antibodies, and (iv) physiological parameters which may be specific to the said patient, such as the rate of clearance of the said antibodies from the blood.

Most preferably, a calibration sample (e.g. a sample with known amounts of ADAs or ASAs) is prepared starting from a body fluid sample initially exempt of the selected labeled polypeptide, and preferably serum or plasma from a non-human mammal or from a human individual, and most preferably human serum or human plasma.

Then, each of the calibration sample is subjected to the same sample preparation steps as that which is described for the test samples elsewhere in the present specification, so as to provide a serial or a set of calibration assay samples (CAS).

For example, the one or more calibration sample(s) may comprise or consist of a spiked sample (e.g. a plasma sample), consisting of a sample in which a known quantity of the analyte (e.g. the ADA or ASA) is present.

Then, each calibration assay sample is subjected to mass spectrometry analysis, and most preferably to a LC-MS/MS analysis, in the same conditions as those described for the test samples elsewhere in the present specification and the values of the mass spectrometry signals generated by a selected surrogate peptide from enzyme proteolysis of the selected labeled polypeptide are recorded.

The individual to be tested may be a mammal or a non-mammal individual, in particular human or non-human. The sample (i.e. liquid, tissue or biopsy) may be any sample susceptible to contain antibodies, in particular ADAs or ASAs, such as blood or a blood-derived sample.

Thus, in some embodiments, the sample which is used in the quantification method originates from a whole human blood sample that has been previously collected from an individual. In preferred embodiments, the blood cells, and especially erythrocytes, are removed by centrifugation so as to obtain a plasma sample. In other preferred embodiments, coagulation of the whole blood sample is allowed to occur and a serum sample is obtained.

In further embodiments, the sample which is used in the detection/quantification method may consist of other extracellular fluids such as lymphatic fluid (endolymph or perilymph) and interstitial fluid.

In some embodiments, the initial sample may be subjected to dilution, e.g. in an aqueous medium such as in a saline solution or in a buffer solution, before being used as the assay sample in the antibody quantification method according to the invention.

However, in the most preferred embodiments, the initial sample, such as a plasma sample or a serum sample, is used without any pre-treatment and in particular is used as such undiluted.

### Proteolysis step

As it is described further herein, the proteolysis step consists of subjecting the mixture (e.g. the solid phase), containing the labeled polypeptide (SIL), to an enzyme proteolysis step (e.g. a protease) so as to generate, notably, proteolysis peptides, namely (i) labeled proteolysis peptides generated from the labeled form of the polypeptide (SIL peptides) capable to be recognized by the said detected or quantified ADA or ASA (ADA peptides or ASA peptides), and the non-labeled proteolysis peptides generated from the non-labeled form of the polypeptide conjugated to the solid support (Internal Standard or IS peptides).

A plurality of embodiments of a proteolysis step may be performed. In particular, the proteolysis enzymes, which may also be termed proteases herein, may be selected in a vast group of proteases well known in the art. Since the cleavage site(s)of each known protease is part of the technical knowledge of the one skilled in the art, the selection of a specific protease for the enzyme proteolysis is correlated to the subsequent monitoring of the expected resulting proteolysis peptides (e.g. the proteotypic peptides) generated therefrom, by mass spectrometry analysis.

In some embodiments of the proteolysis step that are illustrated in the examples herein, the selected protease possesses trypsin activity.

In some other embodiments of the proteolysis step that are illustrated in the examples herein, the selected protease possesses a hinge-targeting activity.

According to these embodiments of the proteolysis step, trypsin is added to the mixture, so as to generate (i) tryptic peptides from the labeled polypeptide bound to ADA/ASA and (ii) tryptic peptides generated by trypsin proteolysis of other non-labeled polypeptides. The specific tryptic peptides derived from the labeled polypeptide may also be termed *"surrogate peptides"* herein, or even *"surrogate tryptic peptides".*

In some embodiments, the enzyme proteolysis is performed by using trypsin as the sole added protease.

The proteolysis step is preferably performed in conditions that are optimal for generating all the expected proteotypic tryptic peptides.

When the protease is trypsin, it may be used a purified trypsin having a low ability to autolysis. Illustratively, it may be used a trypsin termed Trypsin Gold^{®} which is marketed by the company Promega (Madison, WI, United States).

Optimal proteolysis conditions may be reached by using a trypsin / total protein molar ratio ranging from 1:100 to 1:20.

Proteolysis in the presence of trypsin is performed during a period of time that may be optimally adapted.

Advantageously, proteolysis is performed at 37°C during a time ranging from 0.5 hour to 18 hours, for example from 1 hour to 10 hours.

The proteolysis step may be performed at a pH of 6 or more. Further, the proteolysis step may be performed at a pH of less than 8.5, preferably at a pH of 8 or less, which includes at a pH of 7.5 or less, e.g. at a pH of about 7. For trypsin proteolysis, the proteolysis step may be advantageously performed at a pH ranging from about 7.5 to about 8.5.

In most preferred embodiments, the proteolysis step is performed under non-denaturing conditions that is under conditions wherein there is no denaturation of the proteins initially contained in the sample.

In some embodiments, proteolysis is stopped by acidification of the resulting mixture, for example by adding an appropriate acid such as formic acid, so as to decrease the pH of the said resulting mixture below pH 6.

### Proteolysis with a hinge-targeting protease

In some embodiments, in particular when the labeled polypeptide is a labeled form of a therapeutic antibody, proteolysis may be performed by using a hinge-targeting protease. Hinge-targeting proteases are known proteases effecting a cleavage in an antibody protein in the hinge region so as to generate (i) two Fc regions of the heavy chains and (ii) an F(ab')₂ moiety, respectively. Fab moieties may then be obtained from the F(ab')2 moiety, by methods well known form the one skilled in the art, such as by using a reducing agent such as dithiothreitol (DTT).

According to some of those embodiments, the hinge-targeting protease may be selected in a group comprising Gelatinase A (MMP-2) (Tamerius et al., 1975, Int J Cancer, Vol. 16 : 456-464) , Stromyelysin (MMP-3) (Tamerius et al., 1975, Int J Cancer, Vol. 16 : 456-464; Tamerius et al., 1976, J Immunol, Vol. 116 : 724-730; Reichert et al., 2010, Mabs, Vol. 2 : 84-100), Matrilysin (MMP-7) (Tamerius et al., 1975, Int J Cancer, Vol. 16 : 456-464; Tamerius et al., 1976, J Immunol, Vol. 116 : 724-730; Reichert et al., 2010, Mabs, Vol. 2 : 84-100), Gelatinase B (MMP-9) (Reichert et al., 2010, Mabs, Vol. 2 : 84-100), Macrophage metalloelastase (MMP-12) (Tamerius et al., 1976, J Immunol, Vol. 116 : 724-730; Reichert et al., 2010, Mabs, Vol. 2 : 84-100), Collagenase-3 (MMP-13) (Tamerius et al., 1976, J Immunol, Vol. 116 : 724-730), Cathepsin G (Reichert et al., 2010, Mabs, Vol. 2 : 84-100), Pseudolysin (Strohl et al., 2009, Curr Opinion Biotechnol, Vol. 20 : 685-691), Mirabilysin, Glutamyl endopeptidase I (GluV8) (Tamerius et al., 1976, J Immunol, Vol. 116 : 724-730; Reichert et al., 2010, Mabs, Vol. 2 : 84-100), Streptopain (SpeB) (Brezski et al., 2010, mAbs, Vol. 2:3 : 212-220), Trepolisin (Brezski et al., 2010, mAbs, Vol. 2:3 : 212-220) and Immunoglobulin-degrading enzyme from *Streptococcus* (ideS) (Tamerius et al., 1976, J Immunol, Vol. 116 : 724-730; Reichert et al., 2010, Mabs, Vol. 2 : 84-100).

The resulting proteolysis mixture may be collected by centrifugation and/or protein precipitation, before-suspension.

### Quantification of ADAs and ASAs by mass spectrometry

The step of detection and quantification by mass spectrometry is performed according to techniques of that are known in the art.

Preferably, the step is performed according to a Liquid Chromatography coupled to tandem Mass Spectrometry (LC-MS/MS) approach, as it is shown in the examples herein.

According to some embodiments, it is used a triple quadrupole (QqQ) mass spectrometer equipped with an ESI source operating in positive ion mode and using multiple reaction monitoring (MRM) mode for quantification.

According to some embodiments, it is used a Quadrupole-Orbitrap (Q-Exactive) mass spectrometry and/or other High-Resolution Mass Spectrometry (HRMS).

In some embodiments, Liquid Chromatography is performed with a reverse phase chromatography substrate.

Then, in some embodiments, the most abundant state of charge of (i) selected surrogate proteotypic peptides derived from the labeled polypeptides and of (ii) the proteolytic peptides derived from other polypeptides initially present in the test sample are observed preferably between 400 *m*/*z* and 1200 *m*/*z* in ESI ionization source and are selected and fragmented.

At the detection/quantification step by mass spectrometry, Selected Reaction Monitoring (SRM) transitions or Parallel Reaction Monitoring (PRM) specific of the corresponding labeled proteotypic peptide derived from the corresponding labeled polypeptide, are monitored.

The number of selected proteolysis peptides, for which a mass spectrometry signal is determined, may vary according notably to the number of available proteotypic peptides. The number of selected proteotypic peptides for which a mass spectrometry signal is determined may, for example, vary from 1 to 10 proteotypic peptides, depending on the number of proteolysis peptides which are suitable, which encompasses 1, 2, 3, 4, 5, 6, 7, 8, 9 and 10 selected proteotypic peptides.

SRM transitions of selected proteolytic peptides are preferably established after comparing the fragmentation spectra obtained from pure solutions of each of these peptides, with *in silico* fragmentation spectra generated with a relevant available software tool, such as the software commercialized under the name Skyline^{™} by MacCoss Lab Software (USA) and the bioinformatics tool ESP Predictor available from Genepattern (Vincent A. Fusaro, D. R. Mani, Jill P. Mesirov & Steven A. Carr, Nature Biotechnology (2009) 27:190-198), available notably from the Broad Insitute (USA)

For example, detection and quantification of ADAs and ASAs is based on the ratio of the sum of the peak areas of specific SRM transitions of a selected labeled polypeptide and the sum of the peak areas of a reference.

The present invention is further illustrated, without being limited thereto, by the examples below.

### EXAMPLES

The objective of the experiment is to detect and quantify anti-Infliximab and/or anti-Adalimumab ADAs in patient samples using a multiplex ADA Bridging LC-MS assay.

### I. Material & Methods

### Reagents & chemicals

The following commercial anti-drug antibodies (ADA) were used: anti-Infliximab (HCA213 from Bio-Rad) and anti-Adalimumab (HC204 from Bio-Rad), which were spiked at different concentrations in human plasma, from 10 to 500 ng/mL.

### Sample preparation

The strategy is summarized in **figure 3****.** Briefly, it relies on the immobilization of 150 ng of each drug mAb (Infliximab and Adalimumab) in each well of a 96-well MaxiSorp^{™} microplate. The plate is saturated with 5% BSA in PBS for 1h at room temperature and then washed five times with PBST. The sample (100 µL of calibrator or patient plasma) is then added in the well and incubated for 1h at room temperature on an orbital shaker. If ADAs are present, they will recognize their antigen (i.e. the drug mAb coated on the plate). The revelation is performed by the addition of SIL-Infliximab and SIL-Adalimumab (200 ng of each) and an incubation for 1h at room temperature on an orbital shaker. If ADAs have been bound, they will recognize the corresponding SIL-mAb with their second antigen-binding site. An acid elution with a 50% Acetonitrile (ACN)/0.1% Formic Acid (FA) solution allows to release the ADAs and the SIL-mAb (only if ADAs are present). A weak release of the coated drug mAb also occurs and is used as an internal standard both to normalize LC-MS data and as a control for ADA negative samples. After drying with a speed vacuum, trypsin digestion is performed using 50 µL of trypsin at 8 ng/µL and an incubation overnight at 37°C. Digestion was stopped by the addition of 1 µL of 25% formic acid in acetonitrile. Proteotypic peptides are monitored by LC-MS used in MRM mode. The SIL-peptides are only identified in samples with ADAs. The use of calibrators (using commercial ADAs) and the normalization with the unlabeled peptides (ratio heavy/light) allow a reliable quantification of free total ADAs (e.g. all the ADAs not bound to the residual drug mAb putatively present in the patient plasma).

The process is multiplex as the same calibrators allow to perform the calibration curve to detect and quantify both anti-Infliximab and anti-Adalimumab ADAs.

### LC-MS analysis

Proteotypic peptides were monitored by LC-MS used in MRM mode. The SIL-peptides were only identified in samples with ADAs. LC-SRM analyses were performed on a 6500 Qtrap hybrid triple quadrupole/ion trap mass spectrometer (ABSciex, Framingham, MA, USA) equipped with a TurboV source (AB Sciex) and controlled by Analyst software (version 1.6, AB Sciex). The instrument was coupled to an Exion uHPLC system (ABSciex, Framingham, MA, USA). Chromatography was performed using a two-solvent system combining solvent A (0.1% formic acid) and solvent B (100% acetonitrile, 0.1% formic acid). Peptides were separated on a BioZen column 2.6µm Peptide XB-C18 (2.1 × 100 mm) (Phenomenex, Le Pecq, France). Peptide separation was achieved using a multistep gradient from 5% to 25.5% B over 10 minutes at a flow rate of 200 µL/min. MS data were acquired in positive mode with an ion spray voltage of 5500 V and the interface heater temperature was set to 550 °C. Entrance potential (EP) and collision cell exit potential (CXP) were set to 12 and 19 V, respectively. The appropriate Collision Energy (CE) and Declustering Potential (DP) were set for each proteotypic peptide. SRM acquisitions were performed in the scheduled MRM mode with Q1 and Q3 quadrupoles operating at unit resolution, the target scan time was set to 0.7s.

### Data analysis

LC-SRM data were analyzed using Skyline 21.1 [Maclean et al.; "Skyline: an open source document editor for creating and analyzing targeted proteomics experiments; Bioinformatics, 2010, 966-8] Signals with obvious matrix interferences (experiments in biological matrices) were excluded. For absolute quantification of anti-Infliximab and anti-Adalimumab ADAs, labeled/unlabeled peak area ratios were calculated for each SRM transition after verification of coelution profiles.

The use of calibrators (using commercial ADAs) allows to perform calibrations curves using four-parameter logistic (4PL) regression to perform a reliable quantification of free total ADAs (e.g. all the ADAs not bound to the residual drug putatively present in the patient plasma). The process is multiplex as the same calibrators allow to perform the calibration curve to detect and quantify both anti-Infliximab and anti-Adalimumab ADAs [see **figures 4** **and** **5**].

### II. Results.

### Detection & quantification of anti-Infliximab and/or anti-Adalimumab ADAs in human plasma of patients treated with 1 of these 2 drug mAbs.

The objective of the experiment is to detect and quantify anti-Infliximab and/or anti-Adalimumab ADAs in patient samples using a multiplex ADA Bridging LC-MS assay. The proof-of-concept is established on ten plasmas of patients treated with Infliximab and ten with Adalimumab. Each sample has been previously tested in hospital for ADAs using immunological approach. In each case, the selection of plasma includes five samples termed ADA-positive and five samples termed ADA-negative. Each sample is analyzed in a blind test using the ADA-bridging LC-MS assay.

The results are illustrated in **figure 6** for 2 proteotypic peptides: SINSATHYAESVK (or **SEQ ID N°1**) for anti-Infliximab ADAs and APYTFGQGTK (or **SEQ ID N°2**) for anti-Adalimumab ADAs. The results are qualitative (detection of ADAs in the correct samples) and quantitative (in comparison with data from ELISA) over a range of concentrations in human plasma of 20 to 1000 ng/mL of ADAs. One patient treated with Adalimumab, has been previously treated with Infliximab and is ADA-positive for the two antibodies.

### SEQUENCE LISTING

| **SEQ** | **Type** | **Description** | **Sequence** |
|---|---|---|---|
| **1** | Peptide | **Tryptic Proteotypic peptide from Infliximab** | SINSATHYAESVK |
| **2** | Peptide | **Tryptic Proteotypic peptide from Adalimumab** | APYTFGQGTK |
| **3** | Peptide | **Tryptic Proteotypic peptide from Infliximab** | ASQFVGSSIHWYQQR |

## Claims

1. A method for detecting at least one anti-drug antibody (ADA) or anti-self antibody (ASA), in a sample, comprising the steps of:
a) bringing into contact a solid support conjugated to a polypeptide capable of binding to an antigen-binding site of an ADA or ASA with a test sample susceptible to contain the said ADA or ASA, in conditions suitable for the ADA or ASA to bind to the polypeptide of the solid support, whereby a solid phase is provided;
b) bringing into contact the solid support with a labeled form of the polypeptide;
c) performing enzyme proteolysis on the solid phase, or a fraction thereof, whereby a proteolysis sample susceptible to contain one or more proteotypic labeled peptide(s) from the labeled form of the polypeptide is provided;
d) detecting, by mass spectrometry analysis, the proteotypic labeled peptide(s) in the proteolysis sample, thereby detecting the ADA or ASA in the test sample.

2. The method of claim 1, wherein the method is for detecting an Anti-Drug Antibody (ADA).

3. The method of claim 1, wherein the method is for detecting an Anti-Self Antibody (ASA).

4. The method of claim 3, wherein the conjugated polypeptide is an immunogenic protein, or any other polypeptide-based biological active substance suitable to generate an immune reaction; in particular selected from the group consisting of:
- ATG16L1, ABCF2, ABL2, TNK2, EIF2C4, APOL2, APRT, AQP2, ARL6IP4, ARHGEF1, ARHGEF16, ARIH2, ARMC1, ARMC6, ASCC2, ASF1B, ASPH, ASPHD1, ASS1, ATF1, ATG4A, IGK, AZI2,BCL2L12, BCL2L14, HSPC142, C17orf49, BCL11A, BCCIP, BCKDK, BID, BNIP3L, DC42EP4, C7orf27, BYSL, BZW2, C1orf105, C1orf131, CHAF1B, CARD9, CBS, CCDC97, CDC123, CDCA3, CDK6, CENPB, CENPQ, CEP57, VPS24, CHRAC1, C1orf77, C9orf142, CINP, CKAP2, COASY, COG3, COPZ1, CIAPIN1, RCSD1, C18orf25, CRK, CSF1R, CSNK2A1, GPS1, CTDSPL, CTNNAL1, PSCD4, DYNC1LI2, AMD1, GAD2, DCLK1, DDX19B, DDX21, DDX54, DEF6, DGCR8, DHX8, DMD, DNAJC8, LIG3, C19orf26, DPM1, POLE, DPYSL3, DPYSL4, DPYSL5, DTNBP1, DTL, DDX39A, DYNC1H1, ECE1, EIF3S4, ELOVL5, EME1, C11orf30, EPM2AIP1, ETS1, ETS2, FAM125A, MGC40405, C14orf24, C9orf25, FAM63B, FKBP1A, WTAP, FMNL2, FMR1, FKSG44, FYN, GPI, TUBGCP3, SIP1, GGA1,GSK3B, HIST2H2BE, HIST3H2BB, UCHL5IP, HCK, HDAC9, HMGB1, HMGB2, HNRNPK, HSF1, EIF4H, IGF1R, IMPDH2, IFNGR2, ITM2C, JAK1, JAK3, C22orf9, C12orf41, PRKAR2B, CSNK1G3, KDM2A, JMJD2D, KLC2, KLC3, KLHDC4, KLF3, RPS6KA6, RPS6KB2, LARP2, LARP1, LARP4, LARP7, LUC7L2, LCK, LRBA, LRRFIP1, MAP3K2, MAP4K4, MAN2C1, MAGEB2, MAPKAPK2, MARK1, MAX, MBIP, MCF2L, MCM2, MCM6, MED1, MED4, C2orf33, FLJ20323, MAPK6, MAPK13, MKNK2, MAP2K3, CDC42BPA, MSL3L1, MST4, CBFA2T2, MTMR2, MTMR14, MYLK, NADK, NCF1, NFIC, ZCCHC17, NOL7, HSPC111, NPM1, NUP133, NUDT9, OSBPL11, OSBPL1A, PIK3C2A, PCYT1B, PDCD4, PELO, PGM1, PGM2, PLEKHA3, LCP1, PNPT1, PPP1R14A, PSMC4, PSIP1, PSMD4, PSRC1, PTEN, PTPN18, RAB3IP, RAB9A, RASSF3, hCG_20114, RBM39, RBM4B, RAB11FIP2, RING1, RIOK1, RPAIN, RPL12, RSL1D1, RPL30, RPL31, RNF138, ELAC2, ROCK1, POLR2I, POLR3C, RSRC2, MRPS18B, SNRPA1, RUSC1, RUVBL2, SLC23A2, SAE2, SAMHD1, CIP29, SCLT1, SCML1, SELPLG, SENP2, SEPP1, SF3B2, SH2B1, SIN3A, SKIL, SMARCA5, RY1, USP39, SNX1, SP100, SPAG6, SPC24, CCDC99, APEG1, SPHK2, SQSTM1, SFRS6, SSH3, STK33, STK4, STRAP, TSC22D3, GTF2F1, MAP3K7IP1, TAGLN2, TALDO1, TBC1D22A, TBCB, TCF19, SUB1, CCT8, TNS1, TGFBR2, TTN, TPD52L2, TPI1, TRDN, TRPT1, TRPV2, TSGA10, TTC1, TEX264, TXNL4A, TYK2, TYMS, UBE2O, USP15, UCK2, VDP, VAMP4, VAPB, VPS37A, WWC2, WWP2, YES1, ZAP70, LOC730411, CCDC131, ZC3H3, ZHX1, ZNF174, ZNF326, ZRANB2, ANPEP, CCDC90B, DDX43, DPP3, ERR beta-LBD, EXOC5, FTH1, GAGE1, IL10RB, KCNIP2, LSM12, MAP3K14, MAPK14, RUNX1T1, MGC42105, NRBP1, PHYHD1, PLSCR1, RNF7, SGK2, TBN, TAF9B, TPM3, TTYH2, OGN, PIR, FNTB, SERPINB3, LSM3, CSE1L, CSTB, IDH1, TRUB1, ATIC, RFX5, METTL9, S100A7A, SUMO2, PTBP2, ALDOB, GAGE1, CRKL, UBQLN1, EIF1, COQ3, PPP2R1B, GSDML, IL1F8, CTBP2, UBE2V1, GAD1, NUDCD1, UNC119, YKT6, ODF3L2, RWDD4A, GGA3, HPRT1, TCEAL5, SKP1A, ZADH1, AHSA1, PSME2, AGPAT1, C5orf15, ZBED1, C17orf62, UBE2S, RSU1, RAB3IL1, SDF4, ANXA5, PABPC5, SORD, PSMD10, C3orf26, NCF2, UCK1, PITPNB, WARS, DUSP12, COG8, LAIR1, CASP3, AKR1B 10, COQ9, HOXA1, THOP1, GSN, UBA3, GABRA6, SLC22A7, RRM1, EIF2C1, GCC1, USP4, FAM13A1, PFKL, SF3B3, GMFB, C7orf16, MOK, FGF2, aa1-155, SERPINA3, APTX, ARNTL, C20orf43, CYFIP2, CYGB, EIF2B1, FLOT2, GIMAP5, GMDS, HK1, MAGEA4, MAGEA10, MLX, MPP3, NACA2, CYB5R4, NECAP2, PANK1, PDE9A, PIGG, PITPNA, PLCD1, POU5F1, PTMS, DSCR1, RPL22, MRPS27, SAP18, DNTT, TGM4, VSNL1, VPS26A, RCHY1, ZSCAN21, and/or
- Ro-RNP complex, La antigen, Small nuclear RNP, Histones, Topoisomerase I, Centromere, Myeloperoxidase (p-ANCA), proteinase 3 (c-ANCA), Cardiolipin, Citrullinated protein, Carbamylated protein, Rheumatoid Factor (RF), Collagen, Type IV collagen, and/or
- SSA, SSB, Ro52, Ro60, La, RNA-binding proteins, RNP-A, U1-70K, Sm-D3, Jo1, PL7, threonyl t-RNA synthetase, PL-12 alanyl-tRNA synthetase, Mi2 histone acetylase, tRNA synthetase proteins, TIF-γ, MDA-5, 3-hydroxy-3-methylglutaryl-coenzyme A reductase, centromere proteins, CENPA, CENPB, PM/SCL, EXOSC9, EXOS107, RNA polymerase 3 complex, POLR3A, POLR3K, insulin, IA-2, glutamate decarboxylase/GAD65, Znt8, tetraspanin-7, peptidylpropyl isomerase like 2, Mlhl, proteinase-3, myeloperoxidase, nicotinic acetylcholine receptor/nAChR, muscle-associated cell surface tyrosine kinase, low-density lipoprotein receptor-related protein, aquaporin-4, myelin oligodendrocyte glycoprotein, N-methyl-D-asparate receptor/NMDAR, alpha-amino-3-hydroxy-5methyl-4-isoxazolepropionic receptor/AMPAR, leucine rich glioma inactivated 1 protein, neurexin family protein contactin associated protein 2, phospholipase A2 receptor, thrombospondin type-1 domain-containing 7A, GMCSF, interferon-y, CHRNA1, LRP4, GPIHBP1, GluRl, ADAMTS13, FGF23, CSF2RA, IFNGR, PLA2R; and fragments thereof.

5. The method according to claim 2, wherein the conjugated polypeptide is selected from: a mammal antibody, a human antibody, a humanized antibodies, a synthetic antibody, a chimeric antibody, a therapeutic antibody; or a fragment thereof.

6. The method according to claim 2, wherein the conjugated polypeptide is a therapeutic antibody and/or a fusion protein and/or a therapeutic protein selected from: 3F8, Abagovomab, Abatacept, Abciximab, Abituzumab, Abrezekimab, Abrilumab, Actoxumab, Adalimumab, Adecatumumab, Ado-trastuzumab emtansine, Aducanumab, Aflibercept, Afutuzymab, Afasevikumab, Afelimomab, Alacizumab, Alefacept pegol, Alemtuzumab, Alirocumab, Altumomab, Amatixumab pentetate, Amatuximab, Amivantamab, Anatumomab mafenatox, Andecaliximab, Anetumab, Anifromumab ravtansine, Anifrolumab, Ansuvimab, Anrukinzumab, Apolizumab, Aprutumab ixadotin, Arcitumomab, Ascrinvacumab, Aselizumab, Atezolizumab, Atidortoxumab, Atinumab, Altizumab, Atoltivimab, Atorolimumab, Avelumab, Azintuxizumab vedotin, Bamlanivimab, Bapineuzumab, Basiliximab, Bavituximab, BCD-100, Bectumomab, Begelomab, Belatacept, Belantamab mafodotin, Belimumab, Bemarituzumab, Benralizumab, Berlimatoxumab, Bermekimab, Bersanlimab, Bertilimumab, Besilesomab, Bevacizumab, Bezlotoxumab, Biciromab, Bimagrumab, Bimekizumab, Birtamimab, Bivatuzumab, Bleselumab, Blinatumomab, Blontuvetmab, Blosozumab, Bococizumab, Brazikumab, Brentuximab vedotin, Briakinumab, Brodalumab, Brolucizumab, Brontictuzumab, Burosumab, Cabiralizumab, Camidanlumab tesirine, Camrelizumab, Canakinumab, Cantuzumab mertansine, Cantuzumab ravtansine, Caplacizumab, Casirivimab, Capromab, Carlumab, Carotuximab, Catumaxomab, cBR96-doxorubicin immunoconjugate, Cedelizumab, Cemiplimab, Cergutuzumab amunaleukin, Certolizumab pegol, Cetuximab, Cetrelimab, Cetuximab, Cibisatamab, Cirmtuzumab, Citatuzumab bogatox, Cixutumumab, Clazakizumab, Clenoliximab, Clivatuzumab tetraxetan, Codrituzumab, Cofetuzumab pelidotin, Coltuximab ravtansine, Conatumumab, Concizumab, Cosfroviximab, Crenezumab, Crizanlizumab, Crotedumab, CR6261, Cusatuzumab, Dacetuzumab, Daclizumab, Dalotuzumab, Dapirolizumab pegol, Daratumumab, Dectrekumab, Demcizumab, Denintuzumab mafodotin, Denosumab, Depatuxizumab mafodotin, Derlotuximab biotin, Detumomab, Dezamizumab, Dinutuximab, Dinutuximab beta, Diridavumab, Domagrozumab, Dorlimomab aritox, Dostarlimab, Drozitumab, DS-8201, Duligotuzumab, Dupilumab, Durvalumab, Dusigitumab, Duvortuxizumab, Ecromeximab, Eculizumab, Edobacomab, Edrecolomab, Efalizumab, Efungumab, Eldelumab, Elezanumab, Elgemtumab, Elotuzumab, Elsilimomab, Emactuzumab, Emapalumab, Emibetuzumab, Emicizumab, Enapotamab vedotin, Enavatuzumab, Enfortumab vedotin, Enlimomab pegol, Enoblituzumab, Enokizumab, Enoticumab, Ensituximab, Epcoritamab, Epitumomab cituxetan, Epratuzumab, Eptinezumab, Erenumab, Erlizumab, Ertumaxomab, Etanercept, Etaracizumab, Etesevimab, Etigilimab, Etrolizumab, Evinacumab, Evolocumab, Exbivirumab, [fam-]trastuzumab deruxtecan, Fanolesomab, Faralimomab, Faricimab, Farletuzumab, Fasinumab, FBTA05, Felvizumab, Fezakinumab, Fibatuzumab, Ficlatuzumab, Figitumumab, Firivumab, Flanvotumab, Fletikumab, Flotetuzumab, Fontolizumab, Foralumab, Foravirumab, Fremanezumab, Fresolimumab, Frovocimab, Frunevetmab, Fulranumab, Futuximab, Galcanezumab, Galiximab, Gancotamab, Ganitumab, Gantenerumab, Gatipotuzumab, Gavilimomab, Gedivumab, Gemtuzumab ozogamicin, Gevokizumab, Gilvetmab, Gimsilumab, Girentuximab, Glembatumumab vedotin, Golimumab, Gomiliximab, Gosuranemab, Guselkumab, Ianalumab, Ibalizumab, Sintilimab, Ibritumomab tiuxetan, Icrucumab, Idarucizumab, Ifabotuzumab, Igovomab, Iladatuzumab vedotin, Imalumab, Imaprelimab, Imciromab, Imdevimab, Imgatuzumab, Inclacumab, Indatuximab ravtansine, Indusatumab vedotin, Inebilizumab, Infliximab, Intetumumab, Inolimomab, Inotuzumab ozogamicin, Ipilimumab, Iomab-B, Iratumumab, Isatuximab, Iscalimab, Istiratumab, Itolizumab, Ixekizumab, Keliximab, Labetuzumab, Lacnotuzumab, Ladiratuzumab vedotin, Lampalizumab, Lanadelumab, Landogrozumab, Laprituximab emtansine, Larcaviximab, Lebrikizumab, Lecanemab, Lemalesomab, Lendalizumab, Lenvervimab, Lenzilumab, Lerdelimumab, Leronlimab, Lesofavumab, Letolizumab, Lexatumumab, Libivirumab, Lifastuzumab vedotin, Ligelizumab, Loncastuximab tesirine, Losatuxizumab vedotin, Lilotomab satetraxetan, Lintuzumab, Lirilumab, Lodelcizumab, Lokivetmab, Lorvotuzumab mertansine, Lucatumumab, Lulizumab pegol, Lumiliximab, Lumretuzumab, Lupartumab, Lupartumab amadotin, Lutikizumab, Maftivimab, Mapatumumab, Margetuximab, Marstacimab, Maslimomab, Mavrilimumab, Matuzumab, Mepolizumab, Metelimumab, Milatuzumab, Minretumomab, Mirikizumab, Mirvetuximab soravtansine, Mitumomab, Modotuximab, Mogamulizumab, Monalizumab, Morolimumab, Mosunetuzumab, Motavizumab, Moxetumomab pasudotox, Muromonab-CD3, Nacolomab tafenatox, Namilumab, Naptumomab estafenatox, Naratuximab emtansine, Narnatumab, Narsoplimab, Natalizumab, Navicixizumab, Navivumab, Naxitamab, Nebacumab, Necitumumab, Nemolizumab, NEOD001, Nerelimomab, Nesvacumab,Netakimab, Nimotuzumab, Nirsevimab, Nivolumab, Nofetumomab merpentan, Obiltoxaximab, Obinutuzumab, Ocaratuzumab, Ocrelizumab, Odesivimab, Odulimomab, Ofatumumab, Olaratumab, Oleclumab, Olendalizumab, Olokizumab, Omalizumab, Omburtamab, OMS721, Onartuzumab, Ontuxizumab, Onvatilimab, Opicinumab, Oportuzumab monatox, Oregovomab, Orticumab, Otelixizumab, Otilimab, Otlertuzumab, Oxelumab, Ozanezumab, Ozoralizumab, Pagibaximab, Palivizumab, Pamrevlumab, Panitumumab, Pankomab, Panobacumab, Parsatuzumab, Pascolizumab, Pasotuxizumab, Pateclizumab, Patritumab, PDR001, Pembrolizumab, Pemtumomab, Penpulimab, Perakizumab, Pertuzumab, Pexelizumab, Pidilizumab, Pinatuzumab vedotin, Pintumomab, Placulumab, Prezalumab, Plozalizumab, Pogalizumab, Polatuzumab vedotin, Ponezumab, Porgaviximab, Prasinezumab, Prezalizumab, Priliximab, Pritoxaximab, Pritumumab, PRO 140, Quilizumab, Racotumomab, Radretumab, Rafivirumab, Ralpancizumab, Ramucirumab, Ranevetmab, Ranibizumab, Raxibacumab, Ravagalimab, Ravulizumab, Refanezumab, Regavirumab, Regdanvimab, Relatlimab, Remtolumab, Reslizumab, Retifanlimab, Rilonacept, Rilotumumab, Rinucumab, Risankizumab, Rituximab, Rivabazumab pegol, Robatumumab, Rmab, Roledumab, Romilkimab, Romosozumab, Rontalizumab, Rosmantuzumab, Rovalpituzumab tesirine, Rovelizumab, Rozanolixizumab, Ruplizumab, SA237, Sacituzumab govitecan, Samalizumab, Samrotamab vedotin, Sarilumab, Satralizumab, Satumomab pendetide, Secukinumab, Selicrelumab, Seribantumab, Setoxaximab, Setrusumab, Sevirumab, Sibrotuzumab, SGN-CD19A, SHP647, Sifalimumab, Siltuximab, Simtuzumab, Siplizumab, Sirtratumab vedotin, Sirukumab, Sofituzumab vedotin, Solanezumab, Solitomab, Sonepcizumab, Sontuzumab, Sotrovimab, Spartalizumab, Spesolimab, Stamulumab, Sulesomab, Suptavumab, Sutimlimab, Suvizumab, Suvratoxumab, Tabalumab, Tacatuzumab tetraxetan, Tadocizumab, Tafasitamab, Talacotuzumab, Talizumab, Talquetamab, Tamtuvetmab, Tanezumab, Taplitumomab paptox, Tarextumab, Tavolimab, Tebentafusp, Teclistamab, Tefibazumab, Telimomab aritox, Telisotuzumab, Telisotuzumab vedotin, Tenatumomab, Teneliximab, Teplizumab, Tepoditamab, Teprotumumab, Tesidolumab, TGN 1412, Tetulomab, Tezepelumab, TGN1412, Tibulizumab, Tildrakizumab, Tigatuzumab, Timigutuzumab, Timolumab, tiragolumab, Tiragotumab, Tislelizumab, Tisotumab vedotin, TNX-650, Tocilizumab, Tomuzotuximab, Toralizumab, Toripalimab, Tosatoxumab, Tositumomab, Tovetumab, Tralokinumab, Trastuzumab, Trastuzumab duocarmazine, Trastuzumab emtansine, TRBS07, Tregalizumab, Tremelimumab, Trevogrumab, Tucotuzumab celmoleukin, Tuvirumab, Ublituximab, Ulocuplumab, Urelumab, Urtoxazumab, Ustekinumab, Utomilumab, Vadastuximab talirine, Vanalimab, Vandortuzumab vedotin, Vantictumab, Vanucizumab, Vapaliximab, Varisacumab, Varlilumab, Vatelizumab, Vedolizumab, Veltuzumab, Vepalimomab, Vesencumab, Visilizumab, Vobarilizumab, Volociximab, Vonlerolizumab, Vopratelimab, Vorsetuzumab mafodotin, Votumumab, Vunakizumab, Xentuzumab, XMAB-5574, Zalutumumab, Zanolimumab, Zatuximab, Zenocutuzumab, Ziralimumab, Zolbetuximab, Zolimomab aritox; and fragments thereof.

7. The method according to claim 2, wherein the conjugated polypeptide is selected from the group consisting of: Abarelix, Abatacept, Abciximab, Adalimumab, Aflibercept, Agalsidase beta, Albiglutide, Aldesleukin, Alefacept, Alemtuzumab, Alglucerase, Alglucosidase alfa, Alirocumab, Aliskiren, Alpha-1-proteinase inhibitor, Alteplase, Anakinra, Ancestim, Anistreplase, Anthrax immune globulin human, Antihemophilic Factor, Anti-inhibitor coagulant complex, Antithrombin Alfa, Antithrombin III human, Antithymocyte globulin, Aprotinin, Arcitumomab, Asfotase Alfa, Asparaginase, Asparaginase erwinia chrysanthemi, Atezolizumab, Basiliximab, Becaplermin, Belatacept, Belimumab, Beractant, Bevacizumab, Bivalirudin, Blinatumomab, Botulinum Toxin Type A, Botulinum Toxin Type B, Brentuximab vedotin, Brodalumab, Buserelin, C1 Esterase Inhibitor, Canakinumab, Capromab, Certolizumab pegol, Cetuximab, Choriogonadotropin alfa, Chorionic Gonadotropin, Coagulation factor ix, Coagulation factor VIIa, Coagulation factor X human, Coagulation Factor XIII A-Subunit (Recombinant), Collagenase, Conestat alfa, Corticotropin, Cosyntropin, Daclizumab, Daptomycin, Daratumumab, Darbepoetin alfa, Defibrotide, Denileukin diftitox, Denosumab, Desirudin, Digoxin Immune Fab, Dinutuximab, Dornase alfa, Drotrecogin alfa, Dulaglutide, Eculizumab, Efalizumab, Efmoroctocog alfa, Elosulfase alfa, Elotuzumab, Enfuvirtide, Epoetin alfa, Epoetin zeta, Eptifibatide, Etanercept, Evolocumab, Exenatide, Factor IX Complex (Human), Fibrinogen Concentrate (Human), Fibrinolysin aka plasmin, Filgrastim, Filgrastim-sndz, Follitropin alpha, Follitropin beta, Galsulfase, Gastric intrinsic factor, Gemtuzumab ozogamicin, Glatiramer acetate, Glucagon recombinant, Glucarpidase, Golimumab, Gramicidin D, Hepatitis A Vaccine, Hepatitis B immune globulin, Human calcitonin, Human clostridium tetani toxoid immune globulin, Human rabies virus immune globulin, Human Rho(D) immune globulin, Human Serum Albumin, Human Varicella-Zoster Immune Globulin, Hyaluronidase, Ibritumomab, Ibritumomab tiuxetan, Idarucizumab, Idursulfase, Imiglucerase, Immune Globulin Human, Infliximab, Insulin aspart, Insulin Beef, Insulin Degludec, Insulin detemir, Insulin Glargine, Insulin glulisine, Insulin Lispro, Insulin Pork, Insulin Regular, Insulin porcine, Insulin isophane, Interferon Alfa-2a, Interferon alfa-2b, Interferon alfacon-1, Interferon alfa-nl, Interferon alfa-n3, Interferon beta-la, Interferon beta-lb, Interferon gamma-lb, Intravenous Immunoglobulin, Ipilimumab, Ixekizumab, Laronidase, Lenograstim, Lepirudin, Leuprolide, Liraglutide, Lucinactant, Lutropin alfa, Mecasermin, Menotropins, Mepolizumab, Methoxy polyethylene glycol-epoetin beta, Metreleptin, Muromonab, Natalizumab, Natural alpha interferon, Necitumumab, Nesiritide, Nivolumab, Obiltoxaximab, Obinutuzumab, Ocriplasmin, Ofatumumab, Omalizumab, Oprelvekin, OspA lipoprotein, Oxytocin, Palifermin, Palivizumab, Pancrelipase, Panitumumab, Pegademase bovine, Pegaptanib, Pegaspargase, Pegfilgrastim, Peginterferon alfa-2a, Peginterferon alfa-2b, Peginterferon beta-la, Pegloticase, Pegvisomant, Pembrolizumab, Pertuzumab, Poractant alfa, Pramlintide, Preotact, Protamine sulfate, Protein S human, Prothrombin complex concentrate, Ragweed Pollen Extract, Ramucirumab, Ranibizumab, Rasburicase, Raxibacumab, Reteplase, Rilonacept, Rituximab, Romiplostim, Sacrosidase, Salmon Calcitonin, Sargramostim, Satumomab Pendetide, Sebelipase alfa, Secretin, Secukinumab, Sermorelin, Serum albumin, Serum albumin iodonated, Siltuximab, Simoctocog Alfa, Sipuleucel-T, Somatotropin, Somatropin, Streptokinase, Sulodexide, Susoctocog alfa, Taliglucerase alfa, Teduglutide, Teicoplanin, Tenecteplase, Teriparatide, Tesamorelin, Thrombomodulin Alfa, Thymalfasin, Thyroglobulin, Thyrotropin Alfa, Tocilizumab, Tositumomab, Trastuzumab, Tuberculin Purified Protein Derivative, Turoctocog alfa, Urofollitropin, Urokinase, Ustekinumab, Vasopressin, Vedolizumab, Velaglucerase alfa ; and fragments thereof.

8. The method according to any one of the preceding claims, wherein the conjugated polypeptide comprises a variable or hypervariable region of an antibody; in particular a plurality of complementarity-determining regions (CDR) from an antibody.

9. The method according to any one of the preceding claims, wherein the test sample is selected from: a tissue sample, a biofluid, a whole blood sample, a plasma sample and a serum sample; in particular a plasma sample.

10. The method according to any one of the preceding claims, wherein the solid support is selected from: beads, particles, fibers, plates, gels and resins.

11. The method according to any one of the preceding claims, wherein the solid support is conjugated to a plurality of distinct polypeptides capable of binding to one or more ADAs or ASAs which are susceptible to be present in the test sample.

12. The method according to any one of the preceding claims, wherein the labeled form of the polypeptide is a stable-isotopically labeled form of the polypeptide.

13. A method for quantifying at least one anti-drug antibody (ADA) or anti-self antibody (ASA), in a sample, comprising the steps of:
a) bringing into contact a solid support conjugated to a polypeptide capable of binding to an antigen-binding site of an ADA or ASA with a test sample susceptible to contain the said ADA or ASA, in conditions suitable for the ADA or ASA to bind to the polypeptide of the solid support, whereby a solid phase is provided;
b) bringing into contact the solid support with a labeled form of the polypeptide;
c) performing enzyme proteolysis on the solid phase, or a fraction thereof, whereby a proteolysis sample susceptible to contain one or more proteotypic labeled peptide(s) from the labeled form of the polypeptide and one or more proteotypic unlabeled peptide(s) from the conjugated polypeptide is provided;
d) detecting, by mass spectrometry analysis, the proteotypic labeled peptide(s) and proteotypic unlabeled peptide(s) in the proteolysis sample, thereby quantifying the ADA or ASA in the test sample.

14. A kit comprising:
- a solid support conjugated to one or more drug polypeptide(s) capable of binding to an anti-drug antibody (ADA), wherein the one or more drug polypeptide(s) is selected from: a mammal antibody, a human antibody, a humanized antibodies, a chimeric antibody, a synthetic antibody, a therapeutic antibody, an immunogenic polypeptide;
- a stable-isotopically labeled form of the one or more drug polypeptide(s).

15. A kit comprising:
- a solid support conjugated to one or more immunogenic polypeptide(s) capable of binding to an anti-self antibody (ASA);
- a stable-isotopically labeled form of the one or more immunogenic polypeptide(s).

16. The kit according to claim 14 or 15; which further comprises one or more antibodies, or compositions thereof, selected from: an anti-drug antibody (ADA), an anti-self antibody (ASA).
